# EUROPÄISCHE PATENTANMELDUNG

(11) **EP 2 335 691 A1**
(43) Veröffentlichungstag der Anmeldung: **22.06.2011**
(21) Anmeldenummer: 11156648.5
(22) Anmeldetag: 29.04.2005
(51) Int. Cl.: A61K 9/20, A61K 9/28, A61K 31/522, A61K 47/38, A61K 9/00

(54) **Vardenafil Formulierungen mit kontrollierter Wirkstofffreisetzung**

(30) Priorität: 11.05.2004 DE 102004023069
(62) Teilanmeldung aus: 05744781.5
(71) Anmelder: Bayer Schering Pharma AG, 13353 Berlin (DE)
(72) Erfinder: Haning, Helmut Dr., 42327, Wuppertal (DE); Skrabs, Susanne Dr., 13189, Berlin (DE); Serno, Peter Dr., 51467, Bergisch Gladbach (DE); Pauli, Kerstin Dr., 10585, Berlin (DE); Heinig, Roland Dr., 42115, Wuppertal (DE)

(57) **Zusammenfassung**

Die vorliegende Erfindung betrifft neue galenische Darreichungsformen mit kontrollierter Wirkstofffreisetzung, die den PDE 5-Inhibitor Vardenafil und/oder pharmazeutisch verträgliche Salze, Hydrate, Solvate und/oder polymorphe Formen davon als Wirkstoff enthalten, sowie deren Herstellung. Die Erfindung betrifft weiterhin die Anwendung dieser neuen galenischen Darreichungsformen als Arzneimittel und ihre Verwendung zur Herstellung von Arzneimitteln zur Behandlung und/oder Prävention von Erkrankungen bei Menschen und Tieren.

## Beschreibung

Die vorliegende Erfindung betrifft neue galenische Darreichungsformen mit kontrollierter Wirkstoff-freisetzung, die den PDE 5-Inhibitor Vardenafil und/oder pharmazeutisch verträgliche Salze, Hydrate, Solvate und/oder polymorphe Formen davon als Wirkstoff enthalten, sowie deren Herstellung. Die Erfindung betrifft weiterhin die Anwendung dieser neuen galenischen Darreichungsformen als Arzneimittel und ihre Verwendung zur Herstellung von Arzneimitteln zur Behandlung und/oder Prävention von Erkrankungen bei Menschen und Tieren.

Bei dem PDE 5-Inhibitor Vardenafil handelt es sich um die Verbindung der Formel (I) mit dem systematischen Namen {2-Ethoxy-5-[(4-ethyl-1-piperazinyl)sulfonyl]phenyl}-5-methyl-7-propylimidazo[5,1-*ƒ*]triazin-4(3*H*)-on:

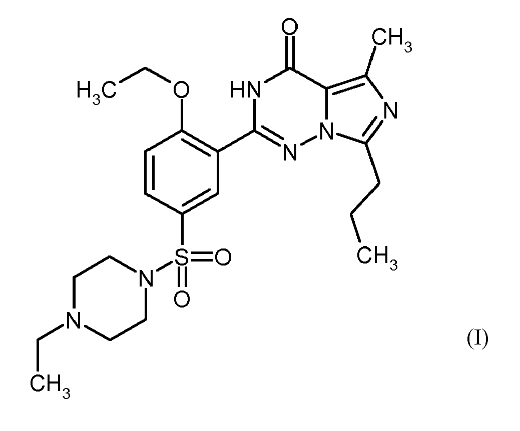

Der intrazelluläre cGMP-Spiegel wird durch das Zusammenspiel von Synthese durch NO-aktivierte Guanylatcyclase einerseits und Abbau durch Phosphodiesterasen (PDEs) andererseits kontrolliert. PDE 5 im Corpus cavernosum-Gewebe des Penis kontrolliert hauptverantwortlich den für die Erektion wichtigen cGMP-Spiegel.

Das NO/cGMP-System spielt eine entscheidende Rolle beim hämodynamischen Prozess der Erektion. Inhibition des cGMP-abbauenden Enzyms PDE 5 ist vor allem in Situationen mit erhöhten NO-Spiegeln, insbesondere bei sexueller Stimulation wirksam. Aufgrund dieser Situation können lang anhaltende Plasmaspiegel eines PDE 5-Inhibitors jeweils bei Auftreten einer sexuellen Stimulation eine Wirkung gegen sexuelle Dysfunktion entfalten. Auch bei anderen Krankheiten kann eine lang anhaltende Exposition eines PDE 5-Inhibitors zu einem verbesserten therapeutischen Effekt, einer reduzierten Fluktuation der Plasmaspiegel, einer Herabsetzung der zu applizierenden Dosis und/oder zu verminderten Nebenwirkungen führen.

Die vorliegende Erfindung betrifft neue galenische Darreichungsformen des PDE 5-Inhibitors Vardenafil, seiner Salze, Hydrate, Solvate, polymorphen Formen und insbesondere des Hydrochlorid-Trihydrats, die sich durch eine kontrollierte Freisetzung des Wirkstoffs auszeichnen.

Vardenafil und seine Herstellung und Verwendung sind zum Beispiel beschrieben in WO 99/24433, WO 02/50076, WO 02/089808 und WO 03/011262.

Darreichungsformen mit kontrollierter Wirkstofffreisetzung sind grundsätzlich im Stand der Technik bekannt. Darreichungsformen mit kontrollierter Wirkstofffreisetzung, die cGMP PDE 5-Inhibitoren enthalten, sind dagegen nur wenig bekannt. Zwar werden in WO 00/24383 pharmazeutische Formulierungen von PDE 5-Inhibitoren mit kontrollierter Freisetzung beansprucht, von diesen PDE 5-Inhibitoren unterscheidet sich Vardenafil jedoch wesentlich aufgrund von speziellen physikochemischen und pharmakokinetischen Eigenschaften. Besonders kritisch ist insbesondere die sehr geringe absolute orale Bioverfügbarkeit von Vardenafil, das einem wesentlich stärkeren first-pass-Effekt unterliegt als andere cGMP PDE 5 Inhibitoren, wie z.B. Sildenafil, und die sehr ausgeprägte pH-Abhängigkeit der Löslichkeit von Vardenafil.

Der bisherige Stand der Technik umfasst lediglich die Anwendung von Vardenafil als schnell freisetzende Tablette zur akuten Behandlung von erektiler Dysfunktion. Die Anwendung von Vardenafil aus Arzneiformen mit kontrollierter Wirkstofffreisetzung ist nicht bekannt und wurde bislang aus verschiedenen Gründen nicht in Betracht gezogen.

Einerseits erfüllten die bislang verwendeten, ausschließlich schnell freisetzenden Arzneiformen des PDE 5-Inhibitors Vardenafil durch ihren schnellen Wirkungseintritt und ihre begrenzte, relativ kurze Wirkdauer das bislang bestehende Zielprofil einer bedarfsorientierten Behandlung, ohne den Patienten unnötig lange der Substanz auszusetzen.

Andererseits wurde befürchtet, dass bei länger andauernder Exposition vermehrt unerwünschte Nebenwirkungen wie z.B. Rückenschmerzen auftreten könnten, wie sie vermehrt bei der Anwendung von PDE 5-Inhibitoren mit langer Eliminationshalbwertszeit beobachtet worden sind.

Es schien bislang somit nicht möglich, Vardenafil als Retardarzneiform einzusetzen, ohne die Sicherheit und Unbedenklichkeit der Arzneimitteltherapie zu beeinträchtigen.

Darüber hinaus wurde aufgrund der physicochemischen und pharmakokinetischen Eigenschaften von Vardenafil, seinen Salzen, Hydraten und Solvaten die Entwicklung und Anwendung einer Retardarzneiform von Vardenafil als nicht möglich erachtet. Die Löslichkeit von Vardenafil, seinen Salzen, Hydraten und Solvaten ist extrem pH-Wert-abhängig: z.B. beträgt die Löslichkeit von Vardenafil hydrochlorid trihydrat in 0,1 N Salzsäure 65 mg/ml (löslich), in Phosphatpuffer 0,15 M pH 4 0,87 mg/ml (sehr schwer löslich) und in Phosphatpuffer 0,15 M pH 7 0,03 mg/ml (praktisch unlöslich). Diese starke pH-Wert-Abhängigkeit stellt insbesondere für die Entwicklung von galenischen Darreichungsformen mit kontrollierter Wirkstofffreisetzung zur oralen Anwendung ein Hindernis dar, da die Arzneiform bei der Passage durch den Gastrointestinal (GI)-Trakt Medien mit stark unterschiedlichen pH-Werten von ca. pH 1 bis pH 7,5 ausgesetzt ist. Um eine ausreichende Bioverfügbarkeit aus einer Retardarzneiform zu erzielen, muss die Absorption einer Substanz möglichst über den gesamten Gastrointestinal-Trakt gegeben sein. Bei Vardenafil war jedoch ein sehr enges Absorptionsfenster zu erwarten, da die Substanz in tieferen Abschnitten des GI-Traktes praktisch unlöslich ist, und somit davon ausgegangen werden musste, dass der Wirkstoff in tieferen Dünndarmabschnitten sowie im Kolon ausfällt und daher aus wesentlichen Bereichen des GI-Traktes nicht absorbiert wird. Außerdem ist bekannt, dass Vardenafil zu 85% im first-pass abgebaut wird und daher eine sehr geringe absolute orale Bioverfügbarkeit von nur 15% besitzt, die deutlich niedriger ist als die absolute orale Bioverfügbarkeit anderer cGMP PDE 5-Inhibitoren. Eine Verlangsamung der Wirkstofffreisetzung führt in der Regel bei Substanzen, die einem sehr hohem first-pass-Effekt, unterliegen, zu einem vollständigen Verlust der oralen Bioverfügbarkeit, da durch das langsame Anfluten des Wirkstoffs die Konzentrationen im Pfortaderblut so niedrig bleiben, dass die Kapazität der metabolisierenden Leberenzyme ausreichend für einen vollständigen Abbau ist.

Aus diesen Gründen war für Vardenafil ein enges Absorptionsfenster und eine unzureichende Bioverfügbarkeit bei Einsatz in Darreichungsformen, die den Wirkstoff kontrolliert über einen größeren Bereich des GI-Traktes freisetzen, zu erwarten, so dass die Entwicklung einer solchen Formulierung praktisch unmöglich erschien.

Eine theoretische Möglichkeit zur Lösung des Problems der unzureichenden Bioverfügbarkeit von Substanzen mit einem engen Absorptionsfenster und bevorzugter Absorption in oberen Abschnitten des GI-Traktes, ist die Verlängerung der Verweilzeit einer Arzneiform im oberen GI-Trakt. Zahlreiche Anstrengungen wurden unternommen, um derartige Darreichungsformen für andere Wirkstoffe zu entwickeln, und inzwischen sind vielfältige Prinzipien bekannt, die eine verlängerte Magenverweilzeit bewirken sollen. Beispielsweise wird in US 6306439 der Einsatz quellender Systeme, die durch Quellung und Volumenausdehnung im Magen verweilen sollen, beschrieben, während EP 0415671 Systeme mit spezieller Geometrie und Größe beansprucht. Bioadhäsive Eigenschaften sollen dagegen z.B. bei den in WO 03/051304 beschriebenen Formulierungen zur Verlängerung der Transit-Zeit genutzt werden. Ein weiteres Prinzip stellen flotierende Arzneiformen mit sehr geringer Dichte dar, wie beispielsweise in US 5626876 beschrieben, aber auch Formulierungen mit hoher Dichte werden zur Verlängerung der Magenverweilzeit beansprucht, wie z.B. in EP 0526862. In der Praxis konnte aber der Erfolg solcher Systeme bislang nicht gezeigt werden. Insbesondere bei Nüchterngabe zeigen derartige Systeme nicht den gewünschten Effekt einer verlängerten Magenverweilzeit, weil die Arzneiformen durch die starken Housekeeper-Waves ohne merkbare Verzögerung aus dem Magen entleert werden.

Aus diesen Gründen bestand der Wunsch nach der Entwicklung einer Arzneiformulierung für Vardenafil, seine Salze, Hydrate, Solvate und polymorphen Formen, durch die die zuvor beschriebenen Probleme des Stands der Technik überwunden werden.

Überraschenderweise ist es nun gelungen, Darreichungsformen zu entwickeln, die den Wirkstoff Vardenafil kontrolliert über einen längeren Zeitraum im gesamten Gastrointestinal-Trakt freisetzen. Es konnten daher Arzneimittelformulierungen mit bestimmten Freisetzungsprofilen gefunden werden, durch die die oben beschriebenen Probleme des Stands der Technik überwunden werden können. Dabei ist entscheidend, dass eine mittlere Freisetzungsrate zwischen 80% in 2 Stunden und 80% in 24 Stunden eingehalten wird.

In klinischen Studien konnte anhand von zahlreichen Formulierungen mit diesen speziellen Freisetzungsprofilen gezeigt werden, dass die Substanz bei Einsatz dieser erfindungsgemäßen Darreichungsformen auch aus tiefen Abschnitten des GI-Traktes absorbiert wird.

Nachdem ursprünglich angenommen war, dass eine schnell freisetzende Formulierung mit begrenzter Expositionszeit die optimale Darreichungsform für Vardenafil zur Therapie der Erektilen Dysfunktion darstellt, wurde nun gefunden, dass eine längere Expositionsdauer deutliche Vorteile hat. Durch eine verlängerte Exposition mit Vardenafil bei Anwendung eines Arzneimittels mit kontrollierter Wirkstofffreisetzung ist es gelungen, das Zeitfenster, in dem verbesserte sexuelle Funktionen erreicht werden können, wesentlich zu verlängern, so dass sexuelle Aktivitäten über einen verlängerten Zeitraum, z.B. bis zu 24 h nach Applikation des Vardenafil-haltigen Arzneimittels ermöglicht werden. Somit wurde eine deutlich verbesserte Flexibilität und Spontaneität im Sexualleben des Patienten erreicht und ein besserer Therapieerfolg sowie eine gesteigerte Zufriedenheit des Patienten erzielt.

Weiterhin sind Darreichungsformen mit kontrollierter Wirkstofffreisetzung des PDE 5-Inhibitors Vardenafil auch für die Therapie von anderen, neuen Indikationen geeignet und zeigen wesentliche Vorteile gegenüber den schnell freisetzenden Arzneiformen des Stands der Technik. Durch die Anwendung der neuen Arzneiformen mit kontrollierter Wirkstofffreisetzung ist es gelungen, wesentlich konstantere Blutspiegel zu erzielen und das Auftreten von Blutspiegelspitzen zu verhindern, wodurch z.B. die therapeutische Wirksamkeit verbessert und die Häufigkeit und Intensität von unerwünschten Nebenwirkungen reduziert werden. Weiterhin erlaubt die Anwendung solcher Darreichungsformen eine Reduktion der Applikationsfrequenz und führt dadurch zu verbesserter Akzeptanz und Compliance beim Patienten.

In den klinischen Studien offenbarte sich erstaunlicherweise außerdem, dass, entgegen der bisherigen, auf dem Stand der Technik basierenden Erwartung, eine Verlängerung der Exposition möglich ist, ohne dass es zu einem Anstieg der Nebenwirkungen, einer Beeinträchtigung der Sicherheit und Unbedenklichkeit bei der Therapie kommt.

Gegenstand der Erfindung sind somit neue galenische Darreichungsformen, die Vardenafil und/oder pharmazeutisch verträgliche Salze, Hydrate, Solvate und/oder polymorphe Formen davon als Wirkstoff enthalten, und eine mittlere Freisetzungsrate zwischen 80% in 2 Stunden und 80% in 24 Stunden aufweisen.

Zur Ermittlung der initialen Freisetzung und der mittleren Freisetzungsrate entsprechend der Definition der Erfindung wird die Wirkstofffreisetzung aus den erfindungsgemäßen Darreichungsformen in der Blattrührer-Apparatur "Apparatur 2" des USP 28-NF23 (The United States Pharmacopoeia USP 28 2005) geprüft. Als Freisetzungsmedium werden 900 ml eines Phosphatpuffers pH 6,8 mit 0,1% (m/V) Natriumlaurylsulfat verwendet (Herstellung von 1 Liter dieses Mediums: 2,747 g Dinatriumhydrogenorthophosphat dihydrat, 0,475 g Citronensäure monohydrat und 10g Natriumlaurylsulfat-Lösung 10% (m/m) werden zu 1000 ml mit entionisiertem Wasser gelöst. Sofern erforderlich wird der pH-Wert mit Natriumhydroxid oder ortho-Phosphorsäure auf 6,8 ± 0,05 eingestellt). Die Freisetzung wird unter Einsatz von Sinkern bei einer Temperatur von 37 ± 0,5°C und einer Umdrehungsgeschwindigkeit des Blattrührers von 75 Umdrehungen pro Minute (UpM) durchgeführt. Aus dem Freisetzungsmedium werden Proben durch eine Filtriereinheit entnommen, die gewährleisten muss, dass Begleitstoffe entfernt werden, und die darin gelöste Wirkstoffmenge durch HPLC mit UV-VIS-Detektion bestimmt. Die so bestimmte Wirkstoffmenge wird in Masse-Prozent der eingesetzten Wirkstoffmenge umgerechnet. Die mittlere Freisetzungsrate im Sinne der vorliegenden Erfindung ist über die Zeit bis zum Erreichen einer Wirkstofffreisetzung von 80% definiert, während die initiale Freisetzung die prozentuale Wirkstofffreisetzung nach 30 Minuten beschreibt.

Die erfindungsgemäßen Darreichungsformen mit kontrollierter Wirkstofffreisetzung besitzen bevorzugt eine mittlere Freisetzungsrate von 80% im Zeitintervall zwischen 3 und 20 Stunden (80% in 3 Stunden und 80% in 20 Stunden).
1. Galenische Darreichungsform mit kontrollierter Wirkstofffreisetzung, die den PDE 5-Inhibitor Vardenafil und/oder pharmazeutisch verträgliche Salze und/oder Hydrate und/oder Solvate davon als Wirkstoff enthalten, und die eine mittlere Freisetzungsrate zwischen 80% in 2 Stunden und 80% in 24 Stunden aufweist.
2. Galenische Darreichungsform nach Anspruch 1 mit einer mittleren Freisetzungsrate zwischen 80% in 3 Stunden und 80% in 20 Stunden.
3. Galenische Darreichungsform nach Anspruch 1 oder 2 mit einer mittleren Freisetzungsrate zwischen 80% in 3 Stunden und 80% in 18 Stunden und einer initialen Freisetzung von weniger als 65% des Wirkstoffs in den ersten 30 Minuten der Freisetzung.
4. Galenische Darreichungsform nach mindestens einem der Ansprüche 1 bis 3, gekennzeichnet durch eine initiale Freisetzung zwischen 0 und 30% des Wirkstoffs in den ersten 30 Minuten der Freisetzung.
5. Galenische Darreichungsform nach mindestens einem der Ansprüche 1 bis 3, gekennzeichnet durch eine initiale Freisetzung zwischen 30 und 60% des Wirkstoffs in den ersten 30 Minuten der Freisetzung.
6. Galenische Darreichungsform nach mindestens einem der Ansprüche 1, 2, 3 oder 4, gekennzeichnet durch eine mittlere Freisetzungsrate zwischen 80% in 4 Stunden und 80% in 18 Stunden und durch eine initiale Freisetzung zwischen 0 und 25% des Wirkstoffs in den ersten 30 Minuten der Freisetzung.
7. Galenische Darreichungsform nach mindestens einem der Ansprüche 1, 2, 3 oder 5, gekennzeichnet durch eine mittlere Freisetzungsrate zwischen 80% in 3 Stunden und 80% in 16 Stunden und durch eine initiale Freisetzung zwischen 35 und 60% des Wirkstoffs in den ersten 30 Minuten der Freisetzung.
8. Galenische Darreichungsform nach mindestens einem der Ansprüche 1 bis 7 zur oralen Anwendung.
9. Galenische Darreichungsform nach mindestens einem der Ansprüche 1 bis 8, gekennzeichnet durch einen Kern, der den Wirkstoff enthält und von einer Membran umschlossen wird, die die Freisetzung des Wirkstoffs kontrolliert.
10. Galenische Darreichungsform nach Anspruch 9, dadurch gekennzeichnet, dass die freisetzungskontrollierende Membran ein filmbildendes Polymer und einen Weichmacher enthält.
11. Galenische Darreichungsform nach Anspruch 9 oder 10, dadurch gekennzeichnet, dass die freisetzungskontrollierende Membran ein filmbildendes Polymer und einen Porenbildner enthält.
12. Galenische Darreichungsform mindestens einem der Ansprüche 9 bis 11, gekennzeichnet dadurch, dass sie Ethylcellulose und/oder Polymethacrylate als filmbildendes Polymer enthält.
13. Galenische Darreichungsform nach mindestens einem der Ansprüche 9 bis 12, gekennzeichnet dadurch, dass der wirkstoffhaltige Kern eine pH-Wert modifizierende Substanz enthält.
14. Galenische Darreichungsform nach mindestens einem der Ansprüche 9 bis 13, gekennzeichnet dadurch, dass es sich bei der pH-Wert modifizierenden Substanz um Bernsteinsäure, Citronensäure, Weinsäure oder Kaliumhydrogentartrat handelt.
15. Galenische Darreichungsform nach mindestens einem der Ansprüche 9 bis 14, gekennzeichnet dadurch, dass die freisetzungskontrollierende Membran ein magensaftresistentes Polymer enthält.
16. Galenische Darreichungsform nach mindestens einem der Ansprüche 1 bis 8, gekennzeichnet durch einen überzogenen Kern, der einen oder mehrere quellbare Hilfsstoffe enthält, die nach Eindringen von Flüssigkeit durch Quellung und Volumenausdehnung den Überzug zum Aufreißen bringen.
17. Galenische Darreichungsform nach mindestens einem der Ansprüche 1 bis 8, gekennzeichnet dadurch, dass sie den Wirkstoff in einer Matrix enthält, die den Wirkstoff durch Diffusion oder Erosion abgibt.
18. Galenische Darreichungsform nach Anspruch 17, gekennzeichnet dadurch, dass die Matrix ein wasserquellbares Polymer umfasst.
19. Galenische Darreichungform nach Anspruch 17 oder 18, gekennzeichnet dadurch, dass es sich um eine Tablette handelt.
20. Galenische Darreichungform nach mindestens einem der Ansprüche 17 bis 19, gekennzeichnet dadurch dass es sich bei dem wasserquellbaren Polymer um Hydroxypropylmethylcellulose oder Hydroxypropylcellulose handelt.
21. Galenische Darreichungsform nach mindestens einem der Ansprüche 17 bis 20, gekennzeichnet dadurch, dass die Matrix eine pH-Wert modifizierende Substanz enthält.
22. Galenische Darreichungsform nach mindestens einem der Ansprüche 17 bis 21 gekennzeichnet dadurch, dass es sich bei der pH-Wert modifizierenden Substanz um Bernsteinsäure, Citronensäure oder Weinsäure handelt.
23. Galenische Darreichungsform nach mindestens einem der Ansprüche 17 bis 22, gekennzeichnet dadurch, dass die Matrix ein magensaftresistentes Polymer enthält.
24. Galenische Darreichungsform nach mindestens einem der Ansprüche 1 bis 8 oder 17, gekennzeichnet dadurch, dass sie ein Schmelzextrudat des Wirkstoffs, das durch Einbettung des Wirkstoffs in eine Matrix mittels eines Schmelzprozesses hergestellt wird, enthält.
25. Galenische Darreichungsform nach Anspruch 24, gekennzeichnet dadurch, dass das Schmelzextrudat ein thermoplastisches Polymer umfasst.
26. Galenische Darreichungsform nach Anspruch 24 oder 25, gekennzeichnet dadurch, dass das Schmelzextrudat ein thermoplastisches Polymer und einen Weichmacher enthält.
27. Galenische Darreichungsform nach mindestens einem der Ansprüche 24 bis 26, gekennzeichnet dadurch, dass es sich bei dem thermoplastischen Polymer um Polyvinylpyrrolidon oder Hydroxypropylcellulose handelt.
28. Galenische Darreichungsform nach mindestens einem der Ansprüche 24 bis 27, gekennzeichnet dadurch, dass das Schmelzextrudat eine pH-Wert modifizierende Substanz umfasst.
29. Galenische Darreichungsform nach mindestens einem der Ansprüche 24 bis 28, gekennzeichnet dadurch, dass das Schmelzextrudat ein magensaftresistentes Polymer enthält.
30. Galenische Darreichungsform nach mindestens einem der Ansprüche 1 bis 8, dadurch gekennzeichnet, dass es sich um eine osmotisches Arzneistofffreisetzungssystem handelt.
31. Galenische Darreichungsform nach Anspruch 30, bestehend aus:
   - einem Kern, der den Wirkstoff, ggf. ein hydrophiles polymeres Quellmittel und ggf.
      einen wasserlöslichen Stoff zum Auslösen der Osmose sowie ggf. weitere pharmazeutisch akzeptable Hilfsstoffe enthält
   - und einer Hülle, die aus einem wasserdurchlässigen, für die Komponenten des wirkstoffhaltigen Kerns undurchlässigen Material besteht und mindestens eine Öffnung aufweist, durch die im Kern vorhandene Bestandteile freigesetzt werden können.
32. Galenische Darreichungsform nach Anspruch 30 oder 31, die Polyethylenoxide, Xanthan Gummi und/oder Copolymere aus Vinylpyrrolidon und Vinylacetat enthält.
33. Galenische Darreichungsform nach mindestens einem der Ansprüche 1 bis 32, die mehrere gleiche oder unterschiedliche Formulierungspartikel wie in den Ansprüchen 9 bis 32 definiert enthält.
34. Galenische Darreichungsform nach mindestens einem der Ansprüche 1 bis 33, die einen Teil des Wirkstoffs in schnell freisetzender Form enthält.
35. Galenische Darreichungsform nach mindestens einem der Ansprüche 1 bis 34, die Vardenafil und/oder Vardenafil in Form seiner Salze, Hydrate, Solvate, Hydrate der Salze und Solvate der Salze sowie der jeweils zugehörigen polymorphen, kristallinen und amorphen Formen enthält.
36. Galenische Darreichungsform nach mindestens einem der Ansprüche 1 bis 35, die zusätzlich mindestens einen anderen Arzneistoff enthält.
37. Galenische Darreichungsform nach mindestens einem der Ansprüche 1 bis 36, die 1 bis 100 mg des Wirkstoffs berechnet als Vardenafil enthält.
38. Galenische Darreichungsform nach mindestens einem der Ansprüche 1 bis 37, die 2 bis 50 mg des Wirkstoffs berechnet als Vardenafil enthält.
39. Galenische Darreichungsform nach mindestens einem der Ansprüche 1 bis 38, die eine Matrix umfasst, die 1 bis 30% (m/m) Vardenafil hydrochlorid trihydrat, 10 bis 65% eines wasserlöslichen Polymers mit einer nominalen Viskosität von mindestens 50 cP und 10 bis 50% (m/m) einer organischen Säure bezogen auf die Gesamtmasse der Matrix enthält.
40. Galenische Darreichungsform nach Anspruch 39, die 2 bis 20% (m/m) Vardenafil hydrochlorid trihydrat, 20 bis 55% eines wasserlöslichen Polymers mit einer nominalen Viskosität von mindestens 50 cP und 20 bis 40% (m/m) einer organischen Säure bezogen auf die Gesamtmasse der Matrix enthält.
41. Galenische Darreichungsform nach Anspruch 40 oder 41 in Form einer Mehrschicht- oder Mantelkern-Tablette, die eine schnell freisetzende Schicht, einen schnell freisetzenden Mantel oder schnell freisetzenden Kern umfasst.
42. Verwendung des PDE 5-Inhibitors Vardenafil, und/oder seiner pharmazeutisch verträglichen Salze und/oder Hydrate und/oder Solvate sowie der zugehörigen polymorphen, kristallinen und amorphen Formen zur Herstellung einer galenischen Darreichungsform wie in den Ansprüchen 1 bis 41 definiert.
43. Verwendung der galenischen Darreichungsformen nach mindestens einem der Ansprüche 1 bis 41 zur Behandlung und/oder Prävention der erektilen Dysfunktion.
44. Verwendung der galenischen Darreichungsform nach mindesten einem der Ansprühe 1 bis 41 zur Behandlung und oder Prävention von Krankheiten, die durch Erhöhung des cGMP-Spiegels einen therapeutischen Nutzen erfahren.

In einer besonders bevorzugten Ausgestaltungsform der Arzneimittelformulierungen mit kontrollierter Wirkstofffreisetzung der vorliegenden Erfindung weist die Formulierung eine mittlere Freisetzungsrate von 80% im Zeitraum von 3 und 18 Stunden und eine initiale Freisetzung von maximal 65% des Wirkstoff in den ersten 30 Minuten der Freisetzung auf.

Die erfindungsgemäßen Darreichungsformen mit kontrollierter Wirkstofffreisetzung können so formuliert sein, dass man eine relative niedrige initiale Freisetzung von 0 bis 30% in den ersten 30 Minuten oder eine relative hohe initiale Freisetzung von 30 bis 60% des Arzneistoffs in den ersten 30 Minuten der Arzneistofffreisetzung erzielt.

In einer bevorzugten Ausführungsform der Darreichungsformen mit kontrollierter Wirkstofffreisetzung der vorliegenden Erfindung mit einer mittleren Freisetzungsrate von 80% im Zeitraum von 4 bis 18 Stunden weist diese eine relativ niedrige initiale Freisetzung von 0 bis 25% in den ersten 30 Minuten der Freisetzung auf.

Eine andere bevorzugte Ausgestaltung der Arzneimittelformulierungen mit kontrollierter Wirkstofffreisetzung besitzt eine mittlere Freisetzungsrate von 80% im Zeitraum von 3 bis 16 Stunden und zeichnet sich durch eine relativ hohe initiale Freisetzung von 35 bis 60% in den ersten 30 Minuten der Wirkstofffreisetzung aus.

Als Darreichungsformen mit kontrollierter Wirkstofffreisetzung dieser Erfindung werden alle Formulierungen bezeichnet, bei denen die Wirkstofffreisetzung so modifiziert ist, dass sie mit einer geringeren Abgaberate erfolgt als aus schnell freisetzenden Arzneiformen, wie z.B. einer konventionellen Tablette oder Kapsel.

Darreichungsformen mit kontrollierter Wirkstofffreisetzung der vorliegenden Erfindung beinhalten auch Formulierungen mit verzögerter Freisetzung, bei der die Abgabe des Wirkstoffs so modifiziert ist, dass die Freisetzung zu einem späteren Zeitpunkt beginnt als bei einer konventionellen schnell freisetzenden Arzneiform. Die anschließende Freisetzung aus einer verzögert freisetzenden Arzneiform kann auch kontrolliert, mit verringerter Freisetzungsrate erfolgen.

Weiterhin umfassen die erfindungsgemäßen Darreichungsformen mit kontrollierter Wirkstofffreisetzung Formulierungen mit pulsatiler Freisetzung, bei denen die Wirkstoffabgabe schubweise zu verschiedenen Zeitpunkten oder an bestimmten Orten des Gastro-Intestinal-Traktes erfolgt, sowie Formulierungen, bei denen verschiedene Prinzipien der kontrollierten Wirkstoffabgabe kombiniert werden.

Darüber hinaus beinhalten die Darreichungsformen dieser Erfindung auch Arzneimittelformulierungen, die einen Teil des Wirkstoffs in schnell freisetzender Form und einen weiteren Teil des Wirkstoffs in kontrolliert freisetzender Form enthalten.

Einen besonderen Aspekt der vorliegenden Erfindung stellen Darreichungsformen mit kontrollierter Wirkstofffreisetzung dar, die Säuren, Basen, Puffersubstanzen und/oder Substanzen mit pH-Wert-abhängiger Löslichkeit, wie z.B. magensaftresistente Polymere, als Zuschlagstoffe enthalten.

Die neuen Formulierungen mit kontrolliertem Freisetzungsverhalten können auf unterschiedlichen Wegen verabreicht werden. Besonders bevorzugt ist die orale Applikation, möglich sind aber auch andere Applikationswege z.B. eine buccale, sublinguale, inhalative, okulare, transdermale oder rektale Verabreichung oder die Anwendung in Form eines Implantates.

Es können feste, halbfeste oder flüssige Formulierungen mit kontrolliertem Freisetzungsverhalten eingesetzt werden. Bevorzugt sind feste Darreichungsformen. Die erfindungsgemäßen Arzneimittelformulierungen können den Wirkstoff in gelöster, suspendierter und/oder fester, amorpher oder kristalliner Form beinhalten.

Zur Herstellung der erfindungsgemäßen Darreichungsformen mit kontrollierter Wirkstofffreisetzung kann der Wirkstoff in verschiedenen Korngrößen, z.B. in ungemahlener, gemahlener oder in mikronisierter Form, eingesetzt werden.

Die zuvor beschriebenen Darreichungsformen mit kontrollierter Wirkstofffreisetzung liegen z.B. in Form von wirkstoffhaltigen Partikeln wie z.B. Pellets, Granulaten, Mikrokapseln, Tabletten, Extrudaten oder Wirkstoffkristallen vor, die mit einer diffusionskontrollierenden Membran überzogen sind. Diese diffusionskontrollierten Systeme sind bevorzugt multipartikulär, d.h. sie bestehen bevorzugt aus einer Vielzahl überzogener Kerne, wie z.B. aus Neutralpellets, auf die eine Mischung des Wirkstoffs mit einem üblichen Binde- und Verdickungsmittel, gegebenenfalls gemeinsam mit üblichen Hilfs- und Trägerstoffen, wie z.B. unten definiert, aufgetragen wird und anschließend mit einem Diffusionslack, der Weichmacher und andere Hilfsstoffe enthalten kann, überzogen werden. Die erfindungsgemäßen diffusionskontrollierten Systeme können außerdem aus homogenen wirkstoffhaltigen Kernen bestehen, die z.B. durch Granulation, Rotorgranulation, Wirbelschichtagglomeration, Tablettierung, Feuchtextrusion oder Schmelzextrusion ggf. mit Spheronisation hergestellt werden und mit einem Diffusionslack, der Weichmacher und andere Hilfsstoffe enthalten kann, überzogen sind. In einer bevorzugten Ausführungsform dieser Erfindung enthalten die wirkstoffhaltigen Partikel Hilfsstoffe wie z.B. Säuren oder Puffersubstanzen, die den pH-Wert modifizieren und dadurch dazu beitragen, die Abhängigkeit der Wirkstofffreisetzung vom pH-Wert des Freisetzungsmediums zu reduzieren. In einer weiteren bevorzugten Ausführungsform dieser Erfindung enthält die diffusionskontrollierte Membran Hilfsstoffe, die durch ihre pHabhängige Löslichkeit die Permeabilität der Membran bei verschiedenen pH-Werten beeinflussen und somit helfen, die pH-Wert-Abhängigkeit der Wirkstofffreisetzung zu minimieren.

Als Binde- und Verdickungsmittel bei der Herstellung von beschichteten Neutralpellets (z.B. bestehend aus Saccharose, mikrokristalliner Cellulose, Citronensäure) werden bevorzugt Hydroxypropylmethylcellulosen (HPMC) und Polyvinylpyrrolidon (PVP) verwendet. Ebenso können andere natürlich, synthetische oder partialsynthetische Polymere wie beispielsweise Methylcellulose (MC), Hydroxypropylcellulose (HPC), andere Hydroxyalkylcellulosen und Hydroxyalkylmethylcellulosen, Carboxymethylcellulosen und deren Salze, Polyacrylsäuren, Polymethacrylate, Gelatine, Stärke oder Stärkederivate eingesetzt werden.

Für die Herstellung von Wirkstoffpellets, wirkstoffhaltigen Partikeln und (Mini-)Tabletten durch Granulation, Wirbelschichtagglomeration, Feuchtextrusion, Tablettierung werden z.B. Cellulose, mikrokristalline Cellulose, Cellulose-Derivate, wie z.B. HMPC, HPC und niedrig substituierte Hydroxypropylcellulose (L-HPC), Dicalciumphosphat, Lactose, PVP und Saccharose als Bindemittel und Füllstoffe eingesetzt.

Schmelzextrusionspellets werden durch Einbettung des Wirkstoffs in thermoplastische Hilfsstoffe wie z.B. HPC, HPMC, Ethylcellulose, Hydroxypropymethylcelluloseacetatsuccinat (HPMCAS), PVP, Vinylpyrrolidon/vinylacetat Copolymer, Polyethylenglycol, Polyethylenoxid, Polymethacrylate, Polyvinylalkohole (PVA), teilverseiftes Polyvinylacetat (PVAc), Polysaccharide (z.B. Alginsäure, Alginate, Galaktomannane) Wachse, Fette und Fettsäurederivate hergestellt.

In einer bevorzugten Ausführungsform dieser Erfindung werden pH-Wert modifizierende Substanzen, wie z.B. Säuren, Basen und Puffersubstanzen, in den wirkstoffhaltigen Kern inkorporiert. Durch Zusatz dieser Stoffe gelingt es, die pH-Wert-Abhängigkeit der Freisetzung von Vardenafil und seinen Salzen, Hydraten, Solvaten deutlich zu reduzieren. Als Hilfsstoffe, die den pH-Wert im wirkstoffhaltigen Kernen modifizieren, kommen beispielsweise in Frage: Adipinsäure, Äpfelsäure, L-Arginin, Ascorbinsäure, Asparaginsäure, Benzolsulfonsäure, Benzoesäure, Bernsteinsäure, Citronensäure, Ethansulfonsäure, 2-Hydroxyethansulfonsäure, Fumarsäure, Gluconsäure, Glucuronsäure, Glutaminsäure, Kaliumhydrogentartrat, Maleinsäure, Malonsäure, Methansulfonsäure, Toluolsufonsäure, Trometamol, Weinsäure. Bevorzugt werden Citronensäure, Bernsteinsäure, Weinsäure, Kaliumhydrogentartrat eingesetzt.

Zur Herstellung des Diffusionslackes eignen sich besonders Ethylcellulosen, z.B. als wässrige Dispersion im Handel unter dem Namen Aquacoat^{®} oder Surelease^{®}, und Polymethacrylate, wie z.B. Eudragit^{®} NE, Eudragit^{®} RS und RL. Aber auch andere Materialien wie z.B. Celluloseacetat und Celluloseacetatbutyrat können als filmbildende diffusionskontrollierende Polymere eingesetzt werden.

In einer bevorzugten Ausführungsform dieser Erfindung enthält der Diffusionslack neben dem diffusionskontrollierenden Polymer auch noch Hilfsstoffe mit pH-abhängiger Löslichkeit, wie z.B. magensaftresistente Polymere wie Cellulosephthalate, insbesondere Celluloseacetatphthalat und Hydroxypropylmethylcellulosephthalat, Cellulosesuccinate, insbesondere Celluloseacetatsuccinat und Hydroxypropylmethylcelluloseacetatsuccinat oder Polymethacrylate (z.B. Eudragit^{®} L). Durch Zusatz dieser Substanzen gelingt es, die Freisetzung von Vardenafil und seinen Salzen, Hydraten, Solvaten bei höheren pH-Werten (z.B. pH 4,5 und pH 6,8) zu beschleunigen und somit die pH-Abhängigkeit der Wirkstofffreisetzung zu reduzieren. Diese Substanzen mit pH-abhängiger Löslichkeit werden in einem Anteil von 0 bis 60% (m/m), bevorzugt 10 bis 50% (m/m) bezogen auf die Masse des Films zugesetzt.

Als Weichmacher werden beispielsweise Citronensäurederivate (z.B. Triethylcitrat, Tributylcitrat, Acetyltriethylcitrat), Phthalsäurederivate (z.B. Dimethylphthalat, Diethylphthalat, Dibutylphthalat), Benzoesäure und Benzoesäureester, andere aromatische Carbonsäureester (z. B. Trimellithsäureester), aliphatische Dicarbonsäureester (z.B. Dialkyladipate, Sebacinsäureester, insbesondere Diethylsebacat, Weinsäureester), Glycerinmono-, Glycerindi- oder Glycerintriacetat, Polyole (z.B. Glycerol, 1,2-Propandiol, Polyethylenglycol unterschiedlicher Kettenlänge), Fettsäuren und Derivate (z.B. Glycerolmonostearate, acetylierte Fettsäureglyceride, Rizinusöl und andere native Öle, Miglyol) und Fettsäurealkohole (z.B. Cetylalkohol, Cetylstearylalkohol) verwendet. Die Art und Menge des Weichmachers werden so gewählt, dass die oben definierte erfindungsgemäße Freisetzung und die erforderliche Stabilität der Arzneiformen erzielt wird. Der Anteil des Weichmachers liegt zweckmäßig bei 0 bis 50% (m/m), bevorzugt bei 0 bis 35% (m/m), besonders bevorzugt bei 0 bis 25% (m/m) bezogen auf die Masse des Films.

Um ein Verkleben der überzogenen Partikel während der Herstellung und im fertigen Produkt zu verhindern, können dem Lack so genannte Antiklebemittel zugesetzt werden, wie z.B. Talkum, Magnesiumstearat, Glycerolmonostearat und Aerosil. Der Anteil dieser Antiklebemittel ist abhängig vom verwendeten Polymer und Weichmacher bzw. Weichmacheranteil und liegt üblicherweise bei 0 bis 50% (m/m) des Gesamtmasse des Lackfilms.

Die erfindungsgemäße Freisetzungsrate wird durch die Lackzusammensetzung und die Dicke der Lackschicht gesteuert. Als Zusätze, die die Permeabilität des Films erhöhen, können so genannte "Porenbildner" in den Lack oder in das zu überziehende Partikel gegeben werden. Als Porenbildner werden lösliche Polymere, wie z.B. Polyethylenglycole, PVP, PVA, HPMC, HPC, Hydroxyethylcellulosen (HEC), MC, Carboxymethylcellulosen oder deren Salze, Dextrine, Maltodextrine, Cylcodextrine, Dextrane oder andere lösliche Substanzen, wie z.B. Harnstoff, Salze (Natriumchlorid, Kaliumchlorid, Ammoniumchlorid, usw.), Zucker (Saccharose, Lactose, Glucose, Fructose, Maltose usw.), Zuckeralkohole (Mannitol, Sorbitol, Xylitol, Lactitol, usw.) verwendet. Bezogen auf die Masse des Diffusionsfilms werden 0 bis 50% (m/m), bevorzugt 0 bis 35% (m/m), besonders bevorzugt 0 bis 20% Porenbildner eingesetzt.

Hilfsstoffe mit pH-abhängiger Löslichkeit, die Bestandteile des Diffusionsfilms sein können, sind z.B. magensaftresistente Polymere wie Cellulosephthalate, insbesondere Celluloseacetatphthalat und Hydroxypropylmethylcellulosephthalat, Cellulosesuccinate, insbesondere Celluloseacetatsuccinat und Hydroxypropylmethylcellulosacetatsuccinat und Polymethacrylate (z.B. Eudragit^{®} L).

Bezogen auf ihre Gesamtmasse bestehen die beschriebenen diffusionskontrollierten Arzneiformen aus 0,5 bis 50% (m/m), bevorzugt 2 bis 40% (m/m) Wirkstoff (berechnet als Vardenafil), 10 bis 95% (m/m) Binde-/Füllmittel bzw. thermoplastischem Hilfsstoff im Falle von Schmelzextrusionspellets, und 5 bis 50 % (m/m), bevorzugt 5 bis 40% (m/m), besonders bevorzugt 5 bis 30% (m/m) Diffusionslack und sie können weitere Zuschlagstoffe (pH-Wert modifizierende Substanzen, andere pharmazeutische übliche Hilfsstoffe) enthalten.

Der Diffusionslack bzw. die Diffusionsschicht enthält bezogen auf die Lackmenge 40 bis 100% (m/m), bevorzugt 50 bis 100% (m/m) Filmbildner (filmbildende diffusionskontrollierende Polymere und ggf. magensaftresistente Polymere), 0 bis 50% (m/m), bevorzugt 0 bis 35% (m/m), besonders bevorzugt 0 bis 25% Weichmacher und 0 bis 50% (m/m), bevorzugt 0 bis 35% (m/m), besonders bevorzugt 0 bis 20% (m/m) Porenbildner (wasserlösliche Polymere und andere wasserlösliche Substanzen) enthält. Außerdem kann der Lack Antiklebemittel, überweise 0 bis 50% (m/m) bezogen auf die Filmmasse, und andere Zuschlagstoffe (Pigmente, Farbstoffe, Tenside, Emulgatoren, andere pharmazeutische übliche Hilfsstoffe) enthalten.

Ein weiterer Aspekt der vorliegenden Erfindung sind überzogene Darreichungsformen, die einen oder mehrere quellbare Hilfsstoffe enthalten, die bei Eindringen von Flüssigkeit durch die Membran stark quellen und durch die Quellung und Volumenausdehnung den Überzug zum Aufreißen bringen. Durch das Aufreißen des Überzugs wird die Arzneistofffreisetzung aus der Darreichungsform ermöglicht und erfolgt in der Regel in pulsatiler Form. Als quellbare Hilfstoffe können diese Formulierungen z.B. Polyvinylpyrrolidone, Crospovidone, vernetzte Natriumcarboxymethylcellulose, vernetzte Natriumcarboxymethylstärke, Polyethylenoxide, Polymethyacrylate, niedrig substituierte Hydroxypropylmethylcellulose (L-HPC) enthalten. Als Lackmaterialien eigenen sich z.B. Celluloseacetat, Ethylcellulose, und Polymethacrylate.

Die beschriebenen überzogenen, diffusionskontrollierten oder pulsatilen Formulierungen können direkt und unverändert als Arzneiform eingesetzt werden. Sie können aber auch, ggf. unter Zusatz von Hilfsstoffen, zur endgültigen Darreichungsform (z.B. Kapsel, Tablette, Sacchetformulierung) weiterverarbeitet werden. Um ein gewünschtes Freisetzungsprofil zu erreichen können auch verschiedene überzogene Formulierungen in einer Arzneiform miteinander kombiniert werden, und eine Verabreichung einer Initialdosis kann beispielsweise durch Kombination mit schnell freisetzenden Formulierungspartikeln, z.B. unlackierten Pellets, Granulaten oder Pulver, erfolgen.

In einer weiteren Ausführung der erfindungsgemäßen Darreichungsformen mit kontrollierter Freisetzung werden Formulierungen verwendet, die den Wirkstoff in einer Matrix umfassen. Diese so genannten Matrixformulierungen setzen den Wirkstoff durch Diffusion und/oder Erosion frei. Bevorzugt liegen diese Formulierungen in der Form einer Tablette oder in Form von mehreren Tabletten, die z.B. verkapselt sein können, vor. Die Tabletten können überzogen oder lackiert sein. Derartige Matrix-Formulierungen werden beispielsweise durch Mischen der Bestandteile und Direkttablettierung, oder durch Trocken- oder Feuchtgranulation mit anschließender Tablettierung hergestellt.

Das Masse-Verhältnis von Wirkstoff zur Gesamtmasse der Matrixformulierung liegt bei diesen neuen Formulierungen im Bereich von 1:1 bis 1:200, vorzugsweise im Bereich 1:2 bis 1:40.

Der Mengenanteil des Matrixbildners liegt bevorzugt im Bereich von 10 bis 70% (m/m) der Masse der Formulierung.

Als Matrixbildner können wasserlösliche, wasserquellbare oder wasserunlösliche Substanzen eingesetzt werden. Bevorzugt enthalten die neuartigen Formulierungen ein oder mehrere wasserquellbare Polymere.

Außerdem bevorzugt sind Arzneizubereitungen im Sinne dieser Erfindung, die wasserlösliche, hydrogelbildende Polymere enthalten, wobei diese Polymere eine nominale Viskosität von mindestens 15 cP, bevorzugt mindestens 50 cP (gemessen als 2%ige wässrige Lösung bei 20°C) haben.

Als wasserlösliche bzw. wasserquellbare matrixbildende Polymere werden bevorzugt Hydroxypropylmethylcellulosen (HPMC), Hydroxyethylmethylcellulosen, Hydroxypropylcellulosen (HPC), Hydroxyethylcellulosen, Methylcellulosen (MC), Ethylcellulosen, andere Alkylcellulosen, Hydroxyalkylcellulosen und Hydroxyalkylmethylcellulosen, Natriumcarboxymethylcellulosen (NaCMC), Alginate, Galaktomannane, wie z.B. Guar und Johannisbrotkernmehl, Xanthane, Polyethylenoxide, Polyacrylsäuren, Polymethyacrylsäuren, Polymethacrylsäure-Derivate, Polyvinylalkohole (PVA), teilverseiftes Polyvinylacetat (PVAc), Polyvinylpyrrolidon (PVP), Agar, Pektin, Gummi arabicum, Traganth, Gelatine, Stärke oder Stärkederivate und Mischungen dieser Substanzen eingesetzt werden.

Besonders bevorzugt ist die Verwendung von HPMC.

Hierbei sollten die erfindungsgemäßen Matrixformulierungen bevorzugt mindestens 10% eines Hydroxypropylmethylcellulosetyps enthalten, dessen nominale Viskosität (gemessen als 2%ige wässrige Lösung bei 20°C) mindestens 15 cP, bevorzugt mindestens 50 cP beträgt. Bevorzugt werden HPMC-Typen mit einem Substitutionsgrad der Methoxygruppen von 16,5-30%, besonders bevorzugt 19-30%, und einem Substitutionsgrad der Hydroxypropoxygruppen von 4-32%, besonders bevorzugt 4-12%, verwendet.

Weiterhin können wasserunlösliche Substanzen als Gerüstbildner in den erfindungsgemäßen Matrixformulierungen eingesetzt werden, z.B. ungesättigte oder gesättigte/hydrierte Fettsäuren sowie deren Salze, Ester oder Amide, Fettsäuremono-, -di- oder -triglyceride, Wachse, Ceramide, Cholesterolderivate und Mischungen dieser Substanzen.

Die Formulierungen der vorliegenden Erfindung können übliche Tablettierhilfsmittel wie z.B. hochdisperses Siliciumdioxid (Aerosil^{®}), Magnesiumstearat, Talkum, PVP, Lactose oder mikrokristalline Cellulose enthalten. Diese liegen im Fall von Lactose und mikrokristalliner Cellulose üblicherweise in einer Menge von 10 bis 50%, in Falle von Mg-stearat zweckmäßig in einer Menge von 0,5 bis 3% und im Falle von Aerosil zweckmäßig in einer Menge von 0,1 bis 2% bezogen auf die Tablettenmasse vor.

In einer besonders bevorzugten Ausführungsformen dieser Erfindung werden Substanzen in die Matrix inkorporiert, die den pH-Wert in der Matrix kontrollieren. Durch den Zusatz solcher pH-Wert modifizierenden Hilfsstoffe und/oder durch den Zusatz von Substanzen, die sich bei steigendem pH-Wert auflösen bzw. aus der Matrix herauslösen und somit die Porosität bzw. Permeabilität der Matrix erhöhen und/oder die Erosion der Matrix fördern, gelingt es, für diese bevorzugten Ausführungsformen der vorliegenden Erfindung eine nahezu pH-Wert-unabhängige Freisetzung zu erzielen.

Als Hilfsstoffe, die den erfindungsgemäßen Matrixformulierungen zum Erzielen einer möglichst pH-Wert-unabhängigen Freisetzung zugesetzt werden können, kommen beispielsweise folgende Substanzen in Frage: Adipinsäure, Äpfelsäure, L-Arginin, Ascorbinsäure, Asparaginsäure, Benzolsulfonsäure, Benzoesäure, Bernsteinsäure, Cellulosephthalate, insbesondere Celluloseacetatphthalat und Hydroxypropylmethylcellulosephthalat, Cellulosesuccinate, insbesondere Celluloseacetatsuccinat und HPMCAS, Citronensäure, Ethansulfonsäure, 2-Hydroxyethansulfonsäure, Fumarsäure, Gluconsäure, Glucuronsäure, Glutaminsäure, Kaliumhydrogentartrat, Maleinsäure, Malonsäure, Methansulfonsäure, Polymethacrylate (z.B. Eudragit^{®}-Typen), Toluolsulfonsäure, Trometamol, Weinsäure. Bevorzugt werden Citronensäure, Bernsteinsäure, Weinsäure, HPMCAS, und Polymethacrylate (z.B. Eudragit^{®} L) eingesetzt. Sofern diese Hilfsstoffe in den erfindungsgemäßen Matrixformulierungen enthalten sind, werden sie typischerweise in einem Anteil von 10 bis 50% (m/m) bezogen auf die Gesamtmasse der Matrix zugesetzt.

Die wirkstoffhaltige Matrix kann auch in speziellen geometrischen Formen vorliegen, bei denen durch die spezielle Geometrie und Matrixoberfläche die Freisetzung beeinflusst wird. Die Matrix-und Freisetzungsoberfläche kann beispielsweise durch Verpressen zu Sonderformaten (z.B. Ringtabletten), und/oder durch Lackieren von Teilflächen oder Aufbringen von Barriereschichten mittels einer Mehrschichtpresse kontrolliert werden.

Formulierungen mit unterschiedlichen Freisetzungseigenschaften können z.B. in Mehrschicht- oder Mantel-Kern-Tabletten zu einer Arzneiform vereinigt werden. So erzielt man beispielsweise durch mehrschichtige Tabletten, die eine schnell freisetzende Schicht umfassen, oder Mantel-Kem-Tabletten mit einem schnell freisetzenden Mantel die erfindungsgemäßen kontrollierten Freisetzungen mit hoher initialer Wirkstofffreisetzung, während man durch Mantel-Kern-Tabletten mit einem schnell freisetzenden Kern eine endbeschleunigte Freisetzung (später Burst) erzielen kann.

Eine andere Ausgestaltungsform der erfindungsgemäßen Darreichungsformen mit kontrollierter Wirkstofffreisetzung ist dadurch charakterisiert, dass der Wirkstoff durch einen Schmelzprozess in eine Matrix bestehend aus einem oder mehreren physiologisch unbedenklichen Hilfsstoffen eingebettet wird. Die Freisetzung des Wirkstoff aus diesen so genannten Schmelzextrudaten erfolgt durch Diffusion und/oder Erosion. Bevorzugt liegen diese Formulierungen in der Form von Granulaten, Pellets oder Tabletten vor. Die durch Schmelzextrusion erhaltenen Formen, insbesondere Pellets und Granulate, können zu anderen Arzneiformen weiterverarbeitet werden, wie z.B. durch Verkapselung oder Tablettierung ggf. unter Zusatz von pharmazeutisch üblichen Hilfsstoffen. Außerdem können die erfindungsgemäßen Schmelzextrudate vermahlen werden und anschließend in dieser zerkleinerten Form zur Herstellung von anderen Darreichungsformen, wie z.B. Matrixtabletten, eingesetzt werden. Die Weiterverarbeitung umfasst auch die Kombination von Formulierungen mit unterschiedlicher Arzneistofffreisetzung, wie z.B. retardiert- und schnell freisetzende Partikel, zu einer Darreichungsform. Die Schmelzextrudate und/oder die Arzneiformen, die aus Schmelzextrudaten hergestellt werden, können überzogen oder lackiert sein.

Die Herstellung der Schmelzextrudate erfolgt durch Mischen des Wirkstoff mit mindestens einem schmelzbaren physiologisch unbedenklichen Hilfsstoff (Träger) und gegebenenfalls weiteren üblichen pharmazeutischen Zuschlagstoffen, Aufschmelzen bei einer Temperatur im Bereich von 50 bis 250°C, vorzugsweise 60 bis 200°C, Spritzgießen oder Extrudieren und Ausformen. Dabei kann das Mischen der Komponenten entweder vor dem Aufschmelzen oder während des Aufschmelzens erfolgen, oder es werden ein Teil der Komponenten aufgeschmolzen und die anderen Bestandteile dieser Schmelze zugemischt. Die Mischung aus dem Trägerstoff, dem Wirkstoff und ggf. vorhandenen Zuschlagsstoffe ist nach Aufschmelzen thermoplastisch verformbar und kann daher extrudiert werden. Zur Ausformung des Gemisches bieten sich zahlreiche Methoden an, beispielsweise Heissgranulierung, Kaltgranulierung, Kalandrierung mit zwei Formwalzen, Extrusion und Verformung des noch plastischen Stranges, z.B. zwischen zwei Bändern oder Walzen, oder das Ausrunden z.B. in einer Luftgranuliereinheit nach Schneiden des Stranges.

Das Masse-Verhältnis von Wirkstoff zur Gesamtmasse des Schmelzextrudates liegt bei diesen neuen Formulierungen im Bereich von 1:3 bis 1:200, vorzugsweise im Bereich 1:4 bis 1:100.

Als thermoplastische Träger, die vorzugsweise in physiologischen Medien quellbar oder löslich sind, kommen beispielsweise in Frage: Polyvinylpyrrolidon (PVP), Copolymerisate von N-Vinylpyrrolidon (NVP) und Vinylestern, insbesondere Vinylacetat, Copolymerisate Vinylacetat und Crotonsäure, teilverseiftes Polyvinylacetat, Polyvinylalkohol, Celluloseester, Celluloseether, insbesondere Methylcellulose und Ethylcellulose, Hydroxyalkylcellulosen, insbesondere Hydroxypropylcellulose, Hydroxyalkylmethylcellulosen, insbesondere Hydroxypropylmethylcellulose und Hydroxyethylmethylcellulose, Carboxymethylcellulosen, Cellulosephthalate, insbesondere Celluloseacetatphthalat und Hydroxypropylmethylcellulosephthalat, Cellulosesuccinate, insbesondere Celluloseacetatsuccinat und Hydroxypropylmethylcelluloseacetatsuccinat, Polyhydroxyalkylacrylate, Polyhydroxyalkylmethacrylate, Polyacrylate und Polymethacrylate (Eudragit^{®}-Typen), Copolymerisate von Methylmethacrylat und Acrylsäure, Polylactide Polyethylenglycole, Polyethylenoxide und Polysaccharide wie Galaktomannane und Alginsäure und deren Alkali- und Ammoniumsalze.

Bevorzugte thermoplastische Hilfsstoffe zur Herstellung der erfindungsgemäßen Darreichungsformen mit kontrollierter Wirkstofffreisetzung sind HPC, PVP, Vinylpyrrolidon/Vinylacetat Copolymere, Polymethyacrylate, insbesondere Eudragit^{®} L HPMCAS, Polyethylenglycole, Polyethylenoxide und deren Mischungen.

Als weichmachende Hilfsstoffe zur Reduktion der Glasübergangstemperatur der Mischung können beispielsweise Propylenglykol, Glycerin, Triethylenglykol, Butandiole, Pentanole, wie Pentaerythrit, Hexanole, langkettige Alkohole, Polyethylenglykole, Polypropylenglykole, Polyethylen-propylenglykole, Silicone, Phthalsäurederivate (z.B. Dimethylphthalat, Diethylphthalat, Dibutylphthalat), Benzoesäure und Benzoesäureester, andere aromatische Carbonsäureester (z. B. Trimellithsäureester), Citronensäurederivate (z.B. Triethylcitrat, Tributylcitrat, Acetyltriethylcitrat), aliphatische Dicarbonsäureester (z.B. Dialkyladipate, Sebacinsäureester, insbesondere Diethylsebacat, Weinsäureester), Glycerinmono-, Glycerindi- oder Glycerintriacetat, Fettsäuren und Derivate (z.B. Glycerolmonostearate, acetylierte Fettsäureglyceride, Rizinusöl und andere native Öle, Miglyol), Fettsäurealkohole (z.B. Cetylalkohol, Cetylstearylalkohol), Zucker, Zuckalkohole und Zuckerderivate (z.B. Erythritol, Isomalt, Lactitol, Mannitol, Maltitol, Maltodextrin, Xylitol) eingesetzt werden. Die Konzentration an Weichmacher liegt üblicherweise bei 0 bis 30% (m/m), vorzugsweise bei 0 bis 20% (m/m) bezogen auf die Gesamtmasse des Schmelzextrudates.

Die extrudierbare Mischung kann neben Wirkstoff, Träger und ggf. Weichmacher noch andere pharmazeutisch übliche Zuschlagstoffe enthalten, beispielsweise Schmier- und Formentrennmittel, Gleit- und Fliessmittel, Füllmittel und Adsorbentien, Stabilisatoren, Radikalfänger, Komplexbildner, Antioxidantien, Photostabilisatoren, Treibmittel, Tenside, Konservierungsmittel, Farb-, Süß- und Geschmacksstoffe.

Voraussetzung für die Eignung einer Substanz als Hilfsstoff sind ausschließlich ausreichende Temperaturbeständigkeit und physiologische Verträglichkeit.

Der Anteil der Zuschlagsstoffe kann bis zu 60% (m/m) der Gesamtmasse des Extrudates betragen.

Schmier- und Formentrennmittel können beispielsweise Stearinsäure und Stearate, insbesondere Aluminium-, Calicium- und Magnesiumstearat, Calciumbehenat, Natriumstearylfumarat, Talkum, Silicone, Wachse, sowie Mono-, Di- und Triglyceride, wie z.B. Glycerolmonostearat, Glyceroldistearate, Glyceroldibehenat, Glyceromonooleat, Glycerylpalmitostearat, in einer Menge von 0 bis 10% (m/m), bevorzugt von 0,5 bis 5% (m/m), bezogen auf die Gesamtmasse des Schmelzextrudates zugesetzt werden.

Als Fliessmittel werden z.B. tierische und pflanzliche Fette, bevorzugt in hydrierter Form und mit einem Schmelzpunkt von mindestens 50°C, Wachse (z.B. Carnaubawachs), Mono-, Di- und Triglyceride (z.B. Glycerolmonostearat, Glyceroldistearate, Glyceroldibehenat, Glyceromonooleat, Glycerylpalmitostearat), Phosphatide, insbesondere Lecithin, in einer Gesamtmenge von 0 bis 30% (m/m), vorzugsweise 0 bis 10% (m/m), bezogen auf die Gesamtmasse des Extrudates verwendet.

Als Füllstoffe kommen Substanzen wie z.B. Titandioxid, Aluminiumoxid, Magnesiumoxid, Kieselsäure und Silikate, Stearinsäure und Stearate, Cellulosederivate (z.B. Methylcellulose), Stärke und Stärkederivate, Zucker, Zuckeralkohole und Zuckerderivate, üblicherweise in einem Anteil von 0 bis 30% (m/m), bevorzugt 0 bis 20% (m/m), bezogen auf die Gesamtmasse des Extrudates zum Einsatz.

Eine bevorzugte Ausführungsform der erfindungsgemäßen Darreichungsformen mit kontrollierter Wirkstofffreisetzung sind Schmelzextrudate, die Hilfsstoffe mit pH-Wert modifizierenden Eigenschaften und/oder pH-Wert-abhängiger Löslichkeit, enthalten. Durch diese Hilfsstoffe (beispielsweise die zuvor bereits mehrfach beschriebenen Säuren, Basen, Puffersubstanzen und magensaftresistenten Polymere), gelingt es, die pH-Wert-Abhängigkeit der Freisetzung von Vardenafil und seinen Salzen, Hydraten, Solvaten zu minimieren.

Bei der Herstellung der Schmelzextrudate kann es zu Ausbildung von so genannten "festen Lösungen" kommen, in denen der Wirkstoff molekulardispers in der Matrix vorliegt.

Eine weitere Ausgestaltung der erfindungsgemäßen Darreichungsformen mit kontrollierter Wirkstofffreisetzung sind osmotische Arzneistofffreisetzungssysteme. Grundsätzlich sind solche osmotischen Systeme im Stand der Technik bekannt. Dabei liegt der Arzneistoffabgabe aus der Arzneiform im Allgemeinen ein osmotischer Druck als treibende Kraft zugrunde. Eine ausführliche Beschreibung der osmotischen Systeme wird z.B. in Verma R.K. et. al. "Osmotic pumps in drug delivery", Critical Reviews™ in Therapeutic Drug Carrier Systems, 21 (2004) 477-520 und Santus G. et al. "Osmotic drug delivery: a review of the patent literature", Journal of Controlled Release 35 (1995) 1-21 gegeben.

Das osmotisches System als Ausführungsform der vorliegenden Erfindung besteht bevorzugt aus:
- einem Kern, der den Wirkstoff, ggf. ein hydrophiles polymeres Quellmittel und ggf. einen wasserlöslichen Stoff zum Auslösen der Osmose sowie ggf. weitere pharmazeutisch akzeptable Hilfsstoffe enthält
- und einer Hülle, die aus einem wasserdurchlässigen, für die Komponenten des wirkstoffhaltigen Kerns undurchlässigen Material besteht und mindestens eine Öffnung aufweist, durch die im Kern vorhandene Bestandteile freigesetzt werden können.

Das Material aus dem die Hülle dieser erfindungsgemäßen Darreichungsformen mit kontrollierter Wirkstofffreisetzung gebildet wird, ist semipermeabel, d.h. durchlässig für Wasser, wässrige Medien und biologische Flüssigkeiten und nicht bzw. sehr wenig permeabel für die Komponenten des Kerns, und geeignet zur Filmbildung. Das selektive semipermeable Hüllmaterial ist unlöslich in Körperflüssigkeiten, erodiert nicht, wird im GI-Trakt nicht abgebaut und unverändert ausgeschieden, oder es zeigt erst gegen Ende der Freisetzungszeit Bioerosion. Typische Materialen für zur Herstellung der Hülle sind literaturbekannt und z.B. in den Patenten US 3916899, US 3977404 und EP 0277092 beschrieben. Beispielsweise können acylierte Cellulosederivate (Celluloseester), die durch Acetylgruppen ein- bis dreifach oder durch Acetylgruppen ein- bis zweifach und einen weiteren von Acetyl verschiedenen Acylrest substituiert sind, verwendet werden, z.B. Celluloseacetat, Cellulosetriacetat, Celluloseacetatethylcarbamat, Celluloseacetatphthalat, Celluloseacetatmethylcarbamat, Celluloseacetatsuccinat, Celluloseacetatdimethylaminoacetat, Celluloseacetatdiethylaminoacetat Celluloseaceatethylcarbonat, Celluloseacetatchloracetat, Celluloseacetatethyloxalat, Celluloseacetatmethylsulfonat, Celluloseacetatbutylsulfonat, Celluloseacetatpropionat, Celluloseacetatoctat, Celluloseacetatlaurat, Celluloseacetat-p-toluolsulfonat, Cellulosaeacetatbutyrat und andere Celluloseacetatderivate sowie Agaracetat und Amyloseacetat. Als semipermeables Membranmaterial eignen sich ferner Ethylcellulose, Copolymere aus Alkylenoxid und Alkyglycidylether, polymere Epoxide, Polyglykole und Polymilchsäurederivate. Außerdem können Mischungen von an sich wasserunlöslichen Acrylaten z.B. ein Copolymerisat von Acrylsäureethylester und Methacrylsäuremethylester eingesetzt werden. Sofern erforderlich kann die Hülle auch Weichmacher, wie z.B. die zuvor bereits genannten weichmachenden Substanzen, und andere Zuschlagsstoffe, wie z.B. Porenbildner, enthalten. Bei Bedarf kann auf die semipermeable Hülle ein Lichtschutzlack aufgebracht werden, der z.B. aus HPMC oder HPC, und einem geeigneten Weichmacher (z.B. Polyethylenglykol) und Pigmenten (z.B. Titandioxid, Eisenoxide) bestehen kann. Um eine Initialdosis des Wirkstoffs verabreichen zu können, kann das osmotische System auch mit einem wirkstoffhaltigen Überzug versehen sein, aus dem der Wirkstoff bei Kontakt mit dem Freisetzungsmedium rasch freigesetzt wird, bevor die osmotisch kontrollierte Abgabe von Wirkstoff aus dem Kern beginnt.

Geeignet als wasserquellbare Polymere, die im Kern enthalten sein können, sind z.B. Polyethylenoxide mit Molekulargewichten von 100.000 bis 8.000.000 (z.B. Polyox^{®}), Xanthan Gummi, Copolymere aus Vinylpyrrolidon und Vinylacetat, Polyvinylpyrrolidone, Crospovidone, vernetzte Natriumcarboxymethylcellulose, vernetzte Natriumcarboxymethylstärke, niedrig substituierte Hydroxypropylmethylcellulose (L-HPC), Poly(hydroxyalkylmethacrylat), Alginate und Galaktomannane sowie weitere in den Patentschriften US 3865108, US 4002173, US 4207893, EP 0052917, EP 0277092 und WO 96/40080 genannte hydrophile polymere Quellmittel und Mischungen daraus.

Als osmotisch aktive Substanzen, die dem Kern zur Induzierung der Osmose zugesetzt werden können, sind prinzipiell alle wasserlöslichen, physiologisch unbedenkliche Stoffe geeignet, wie z.B. die in den Pharmocopöen und in "Remingtons Pharmaceutical Science" genannten wasserlöslichen Substanzen. Insbesondere können wasserlösliche Salze von anorganischen und organischen Säuren oder nicht-ionische organische Stoffe mit hoher Wasserlöslichkeit, wie z.B. Kohlenhydrate, insbesondere Zucker, oder Aminosäuren eingesetzt werden. Im folgenden sind beispielhaft einige Substanzen genannt, die einzeln oder als Mischung zur Induzierung der Osmose in den Kern inkorporiert werden können: anorganische Salze wie Choride, Sulfate, Sulfite, Carbonate, Bicarbonate, Phosphate, Hydrogenphosphate und Dihydrogenphosphate der Alkali- und Erdalkalimetalle, wie z.B. Natrium, Lithium, Kalium, Calcium oder Magnesium, organische Säuren wie Adipinsäure, Ascorbinsäure, Bernsteinsäure, Citronensäure, Fumarsäure, Maleinsäure, Weinsäure, Benzoesäure sowie deren Alkali- oder Erdalkalisalze, Acetate, Pentosen, wie z.B. Arabinose, Ribose oder Xylose, Hexosen, wie Glucose, Fructose, Galaktose oder Mannose, Disaccharide wie Saccharose, Maltose oder Lactose, Trisaccharide, wie Raffinose, Zuckeralkohole, wie Mannitol, Sorbitol, Maltitol, Xylitol oder Inositol, und Harnstoff.

Besonders bevorzugt werden Natriumchlorid und Natriumbicarbonat verwendet.

Darüber hinaus kann das osmotische System andere pharmazeutisch übliche Zuschlagstoffe, wie z.B. Schmier- und Formentrennmittel, Gleitmittel, Bindemittel, Farbstoffe, Verdickungsmittel, Schutzkolloide, Stabilisatoren und Tenside enthalten.

Die Herstellung des erfindungsgemäßen osmotischen Freisetzungssystems erfolgt mit Hilfe von Standardtechniken wie Feuchtgranulation oder Trockenkompaktierung und Tablettierung zur Herstellung des wirkstoffhaltigen Kerns und anschließende organische Lackierung.

Die Hülle des osmotischen Systems weist mindestens eine Austrittsöffnung auf, durch die der Wirkstoff, ggf. zusammen mit anderen Bestandteilen des Kerns, freigesetzt wird. Die Öffnung kann auf verschiedene Weisen in die Hülle eingebracht werden, z.B. durch Stanzen, mechanisches Bohren oder mittels eines Laserbohrers. Die Bezeichnung "Öffnung" umfasst auch bioerodierbare Materialien, die sich bei Anwendung dieser erfindungsgemäßen Darreichungsform aus der Hülle herauslösen und somit in situ zur Ausbildung von Austrittsöffnungen führen. Die Beschaffenheit und Herstellung der Öffnungen sind im Stand der Technik bekannt und z.B. in den Patenten US 3485770, US 3916899, US 4063064 und US 4088864 erläutert.

Die erfindungsgemäße Freisetzungsrate wird in erste Linie durch die Zusammensetzung und Dicke der semipermeablen Hülle, durch Art und Menge des ggf. vorhandenen polymeren Quellmittel und durch die Art und Menge des ggf. vorhandenen, osmotischen aktiven Stoffes, der zur Induzierung der Osmose dient, eingestellt.

In einem weiteren Aspekt der Erfindung kann es sich um eine Formulierung handeln, in der der Wirkstoff als Ionenaustauscher-Komplex vorliegt.

Mehrere Partikel der oben genannten Formulierungsprinizipien können zusammen in einer Darreichungsform vorliegen (z.B. Kapsel gefüllt mit mehreren wirkstoffhaltigen Matrices). Darüber hinaus können auch mehrere der verschiedenen Ausführungsformen (z.B. Pellets mit Diffusionslack und Matrixtablette) in einer Arzneiform kombiniert werden.

Gegenstand der vorliegenden Erfindung ist weiterhin die Kombination von Formulierungen mit unterschiedlichen Freisetzungseigenschaften, z.B. schnell freisetzend und retardiert freisetzend, in einer Arzneiform.

Die erfindungsgemäßen Arzneiformen können überzogen und lackiert sein, z.B. um Lichtschutz zu erzielen, Geschmacksmaskierung zu erreichen oder den Ort bzw. Zeitpunkt des Beginns der Arzneistofffreisetzung zu kontrollieren.

Die erfindungsgemäße Darrreichungsform mit kontrollierter Wirkstofffreisetzung ist bevorzugt eine Formulierung, bei der der maximale Blutspiegel (cₘₐₓ) nach Applikation im Vergleich zu einer schnell freisetzenden Arzneiform gleicher Dosierung reduziert ist und bei der die mittlere Verweilzeit (Mean residence time, MRT) des Arzneistoffs im Körper gegenüber einer schnell freisetzenden Arzneiform verlängert ist.

Die Bestimmung der pharmakokinetischen Parameter AUC, tₘₐₓ, cₘₐₓ und MRT erfolgt wie in Gibaldi M., Perrier D. "Pharmacokinetics", 2nd edition. Marcel Dekker, New York, 1982 und in Rowland M., Tozer T.N. "Clinical Pharmacokinetics: Concepts and Applications", Lea & Febiger, Philadelphia, 1980 beschrieben.

Die vorliegende Erfindung umfasst auch die Verwendung der neuen galenischen Darreichungsformen zur Herstellung von Arzneimitteln, die zur Behandlung und/oder Prävention von Erkrankungen bei Menschen und Tieren bestimmt sind.

Im Allgemeinen hat es sich als vorteilhaft erwiesen, bei oraler Applikation am Menschen eine Wirkstoffdosis von etwa 1 bis 100 mg, vorzugsweise von etwa 2 bis 50 mg zu verabreichen. Die Dosierung der neuen Darreichungsformen kann auch einschleichend, d.h. über einen längeren Zeitraum (zum Beispiel 2-10 Tage) mit progressiv steigender Dosis erfolgen. Die Behandlung mit der neuen Formulierung kann auch an mehreren aufeinander folgenden Tagen, beispielsweise täglich oder in einem anderen festen zeitlichen Rhythmus erfolgen.

Die erfindungsgemäßen neuen Darreichungsformen sind geeignet zur Prophylaxe und/oder Behandlung von Erkrankungen, bei denen ein Anstieg der cGMP-Konzentration heilsam ist, d.h. Erkrankungen, die im Zusammenhang mit cGMP-regulierten Vorgängen stehen (im Englischen meist einfach als 'cGMP-related diseases' bezeichnet).

Die neuen Darreichungsformen des PDE 5-Inhibitors Vardenafil mit kontrollierter Freisetzung können in Arzneimitteln zur Behandlung von kardiovaskulären Erkrankungen wie beispielsweise zur Behandlung und/oder Prophylaxe von Bluthochdruck, neuronaler Hypertonie, stabiler und instabiler Angina, peripheren und kardialen Gefäßerkrankungen, von Arrhythmien, zur Behandlung von thromboembolischen Erkrankungen und Ischämien wie Myokardinfarkt, cerebralen Ischämien, transiente ischämische Attacken, Angina pectoris, primäre pulmonale Hypertension, sekundäre pulmonale Hypertension, pulmonale arterielle Hypertension, portopulmonale Hypertension, hepatopulmonales Syndrom, durch Medikamente wie Amphetamine ausgelöste pulmonale Hypertonie, "interstitial lung disease", mit HIV auftretende pulmonale Hypertension, thromboembolische pulmonale Hypertension, pulmonale Hypertension bei Kindern und Neugeborenen, durch atmosphärische Hypoxie ausgelöste pulmonale Hypertension (Höhenkrankheit), COPD, Emphysem, chronisches Asthma, Mucoviszidose bedingte pulmonale Hypertonie, Rechtsherzinsuffizienz, Linksherzinsuffizienz und Globalinsuffizienz, periphere Durchblutungsstörungen, zur Verhinderung von Restenosen nach Thrombolysetherapie, percutaner transluminaler Angioplastie (PTA), percutaner transluminaler Koronarangioplastie (PTCA), Bypass, für die Behandlung cerebrovaskulärer Erkrankungen, für die Behandlung und/oder Prophylaxe von Erkrankungen des Urogenitalsystems wie Prostatahypertrophie, Inkontinenz sowie insbesondere zur Behandlung und/oder Prophylaxe der erektilen Dysfunktion, vorzeitiger Ejakulation, der benignen Prostatahyperplasie, der weiblichen sexuellen Dysfunktion und der weiblichen sexuellen Erregungsstörung eingesetzt werden.

Darüber hinaus betrifft eine weitere Ausführungsform der Erfindung die Verwendung der neuen Darreichungsform des PDE 5-Inhibitors Vardenafil mit kontrollierter Wirkstofffreisetzung zur Herstellung eines Arzneimittels zur Behandlung und/oder Prophylaxe von Störungen der Wahrnehmung, Konzentrationsleistung, Lernleistung und/oder Gedächtnisleistung, insbesondere wenn die Störung eine Folge von Demenz ist. Besonders eignen sich die erfindungsgemäßen neuen Formulierungen zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierten Lern- und Gedächtnisstörungen, altersassoziierten Gedächtnisverlusten, vaskulärer Demenz, Schädel-Hirn-Trauma, Schlaganfall, nach Schlaganfällen auftretender Demenz ("post stroke dementia"), posttraumatischem Schädel-Hirn-Trauma, allgemeinen Konzentrationsstörungen, Konzentrationsstörungen bei Kindern mit Lern- und Gedächtnisproblemen, der Alzheimer'schen Krankheit, Demenz mit Lewy-Körperchen, Demenz mit Degeneration der Frontallappen einschließlich des Pick's Syndroms, der Parkinson'schen Krankheit, Progressive nuclear palsy, Demenz mit corticobasaler Degeneration, Amyolateralsklerose (ALS), der Huntington'schen Krankheit, multipler Sklerose, thalamischer Degeneration, Creutzfeld-Jacob-Demenz, HIV-Demenz, Schizophrenie mit Demenz oder Korsakoff-Psychose.

Die neuen Darreichungsformen des PDE 5-Inhibitors Vardenafil können auch zur Behandlung und/oder Prophylaxe von Psoriasis, Krebs, Blasenerkrankungen, Nitrat-induzierter Toleranz, Präeklampsie, Alopecia, Schmerz, Gehörsturz, Tinnitus oder dem renalen Syndrom eingesetzt werden.

Die neuen Formulierungen des PDE 5-Inhibitors Vardenafil können auch zur Behandlung und/oder Prophylaxe von Augenerkrankungen wie Glaukom, von zentraler retinaler oder posteriorer cilliarer Arterienokklusion, zentraler retinaler Venenokklusion, optischer Neuropathie wie anteriorer ischämischer optischer Neuropathie und glaukomatöser optischer Neuropathie sowie von makulärer Degeneration dienen.

Die neuen Formulierungen des PDE 5-Inhibitors Vardenafil können ebenfalls zur Herstellung von Arzneimitteln zur Behandlung und/oder Prophylaxe von koronarer Herzkrankheit, Diabetes, Insulin-Resistenz, Hyperglykämie, Pancreatitis, diabetischer Gastroparese, diabetischer Nephropathie, diabetischer Neuropathie, diabetischer Retinopathie, diabetischem Gangrän, diabetischer Glomerulosklerose, diabetischer Dermopathie, diabetischer Arthropathie, diabetischem Katarakt, zur Behandlung von Störungen der Peristaltik von Magen und Speiseröhre, von Osteoporose, weiblicher Infertilität, vorzeitigen Wehen, Leberzirrhose, akutem und chronischem Nierenversagen, zystischer Fibrose, Bronchitis und allergischer Rhinitis verwendet werden.

Die neuen Formulierungen des PDE 5-Inhibitors Vardenafil können auch zur Behandlung und/oder Prophylaxe von kardialer Ischämie, zur Erzielung oder Verbesserung eines "Preconditioning"-Effekts, zur Behandlung eines akuten Myokardinfarktes und von Reperfusionsschäden, speziell nach einem Myokardinfarkt, zur Behandlung männlicher Unfruchtbarkeit, des Raynaud's Syndroms, von Claudicatio intermittens, der Peyronie-Krankheit, zur Behandlung von fibrotischen Erkrankungen, von Arteriosklerose, zur Verbesserung der Spermien-Motilität, zur Behandlung von Depression, Leukämie (z.B. der chronischen lymphozytischen Leukämie), zur Behandlung von Priapismus, zur Behandlung der Plättchenadhäsion und -aggregation bei renaler Ischämie, zur Unterstützung und Förderung der Leberregeneration nach chirugischer Leberresektion oder bei Leberkrebs, zur Inhibition der Kontraktion der Ösophagusmuskulatur (z.B. bei Nussknacker-Ösophagus oder Ösophagospasmen), zur Behandlung von Achalasie, weiblicher Unfruchtbarkeit und Dysmenorrhoe, zur Behandlung von Lebererkrankungen wie z.B. Leberzirrhose, zur Behandlung von Lupus, hypertensivem systemischen Lupus erythematodes, Sklerodermia, zur Behandlung von multipler Sklerose, rheumatoider Arthritis, Allergie, Autoimmun-Erkrankungen, Osteoporose, Kachexie, polycystischem Ovarien-Syndrom, entzündlichen Darmerkrankungen wie z.B. Morbus Crohn und Colitis ulcerosa, Hyperlipidämie und Dyslipidämie, zur Wachstumsförderung und Überlebensverbesserung von Oozyten, Zygoten, Embryos oder Föten, zur Gewichtssteigerung bei Frühgeburten, zur Steigerung der Milchproduktion bei Säugetieren, speziell beim Menschen, zur Behandlung von Migräne, Inkontinenz, akutem und chronischem Nierenversagen, der glomerulären Erkrankung, von Nephritis, Tubulointerstitial-Erkrankungen, Glomuleropathie, Haarverlust, Amnesie, Bewusstseinsstörungen, Autismus, Sprachstörungen, Lennox-Syndrom und Epilepsie eingesetzt werden.

Außerdem verstärkt die Anwendung der neuen erfindungsgemäßen Formulierungen die Wirkung von Substanzen, wie beispielsweise EDRF (Endothelium derived relaxing factor), ANP (atrial natriuretic peptide), von Nitrovasodilatoren und allen anderen Substanzen, die auf eine andere Art als Phosphodiesterase-Inhibitoren die cGMP-Konzentration erhöhen.

Die neuen Darreichungsformen des PDE 5-Inhibitors Vardenafil können auch in Kombination mit anderen Arzneimittel-Wirkstoffen angewendet werden. Bevorzugt sind dabei beispielsweise Inhibitoren der HMG-CoA-Reduktase (z.B. Simvastatin, Atorvastatin, Fluvastatin, Rosuvastatin, Pravastatin, Itavastatin), CETP-Inhibitoren (z.B. Torcetrapib, JTT-705), ACE-Inhibitoren (z.B. Enalapril, Captopril, Benazepril, Cilazapril, Fosinopril, Quinapril, Lisinopril, Ramipril), PPARalpha-Agonisten (z.B. Fenofibrat, Bezafibrat, GW 590735), PPARgamma-Agonisten (z.B. Rosiglitazon), Aldose-Reduktase-Inhibitoren, Ezetimibe, Thrombozytenaggregationshemmer (z.B. Aspirin, Clopidogrel, Ticlopidin, Dipyridamol), Thrombin-Inhibitoren (z.B. Ximelagatran, Melagatran, Bivalirudin, Clexane), beta-Blocker (z.B. Propanolol, Atenolol), Diuretika (z.B. Furosemid), Insulin und Insulinderivate sowie oral wirksame hypoglykämische Wirkstoffe. Insulin und Insulinderivate umfasst hierbei sowohl Insuline tierischen, menschlichen oder biotechnologischen Ursprungs als auch Gemische hieraus. Weiterhin können die neuen Darreichungsformen des PDE 5-Inhibitors Vardenafil auch in Kombination mit Sulphonylharnstoffen (z.B. Tolbutamid, Glibenclamid, Glimepirid, Glipizid oder Gliclazid), Biguanid-Derivaten (z.B. Metformin), alpha-Glucosidase-Inhibitoren (z.B. Miglitol oder Acarbose), Meglitiniden (z.B. Repaglinid, Nateglinid), Wirkstoffen gegen Obesitas (z.B. Orlistat, Sibutramin), GPIIb-IIIa-Antagonisten (z.B. Tirofiban, Abciximab), Faktor Xa-Inhibitoren (z.B. DX 9065a, DPC 906, JTV 803, BAY 597939), Calciumantagonisten (z.B. Nifedipin, Amlodipin, Verapamil, Diltiazem), Antagonisten der alpha1-Rezeptoren, Angiotensin AII-Antagonisten (z.B. Candesartan, Losartan, Valsartan, Telmisartan), anderen PDE 5-Inhibitoren (z.B. Sildenafil, Tadalafil) oder anderen Wirkstoffen zur Behandlung der erektilen Dysfunktion (z.B. Apomorphin) eingesetzt werden.

Zur Herstellung der neuen Formulierungen des PDE 5-Inhibitors Vardenafil können neben dem Hydrochlorid-Trihydrat und dessen polymorphen, kristallinen und amorphen Formen auch andere physiologisch unbedenkliche Salze von Vardenafil sowie Vardenafil selbst verwendet werden. Physiologisch unbedenkliche Salze können Salze von Vardenafil mit anorganischen oder organischen Säuren sein. Bevorzugt sind Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Citronensäure, Weinsäure, Milchsäure, Benzoesäure, oder Methansulfonsäure, Ethansulfonsäure, Phenylsulfonsäure, Toluolsulfonsäure oder Naphthalindisulfonsäure. Die Verwendung dieser Salze und ihrer polymorphen, kristallinen und amorphen Formen sowie die Verwendung der polymorphen, kristallinen und amorphen Formen von Vardenafil zur Herstellung der neuen Darreichungsformen mit kontrollierter Wirkstoff-Freisetzung sind ebenfalls Gegenstand der vorliegenden Erfindung.

In der erfindungsgemäßen neuen Formulierung können Vardenafil und seine Salze auch als Hydrate vorliegen. Im Rahmen der Erfindung werden unter Hydraten solche Verbindungen verstanden, die im Kristall Wasser enthalten. Solche Verbindungen können ein oder mehrere, typischerweise ein bis sechs Äquivalente Wasser enthalten. Hydrate lassen sich beispielsweise herstellen, indem man die betreffende Verbindung aus Wasser oder einem wasserhaltigen Lösungsmittel kristallisiert.

In der neuen Formulierung der vorliegenden Erfindung können Vardenafil und seine Salze auch als Solvate vorliegen. Im Rahmen der Erfindung werden unter Solvaten solche Verbindungen verstanden, die im Kristall physiologisch verträgliche Lösungsmittel enthalten.

Die nachfolgenden Beispiele dienen zu Veranschaulichung der vorliegenden Erfindung, ohne sie zu begrenzen:

### Beispiel 1: Diffusionspellets

### a) Herstellung der wirkstoffbeschichteten Pellets

| **Einsatzstoffe** | Zusammensetzung |
|---|---|
| | in mg/20 mg |
| Vardenafil hydrochlorid trihydrat mikronisiert (Vardenafil HCl trihydrat mikronisiert)^{a} | 23,7 |
| Kaliumhydrogentartrat | 40,0 |
| Neutralspellets^{b} | 29,3 |
| HPMC 15 cP | 7,0 |
| (Wasser)^{c} | (200) |

| | |
|---|---|
| ^{a} Wirkstoffmenge entsprechend 20 mg Vardenafil ^{b} Saccharosepellets ^{c} Hilfsstoff wird während des Prozesses entfernt; Menge abhängig von Ansatzgröße | |

Die Neutralpellets werden mit einer Dispersion bestehen aus dem mikronisierten Wirkstoff, HPMC, Kaliumhydrogentartrat und Wasser in einem Wirbelschichtgranulator mit Wurstereinsatz, beschichtet.

### b) Lackierung der Pellets

| **Einsatzstoffe** | Zusammensetzung |
|---|---|
| | in mg/20 mg |
| Wirkstoffpellets | 100,0 |
| Ethylcellulose^{a} | 6,0 |
| HPMCAS^{b} | 4,0 |
| Triethylcitrat | 2,0 |
| (Wasser)^{c} | (88,0) |

| | |
|---|---|
| ^{a}Trockensubstanz von Aquacoat ECD 30 ^{b}Hydroxypropylmethylcelluloseacetatsuccinat Aqoat AS-LF ^{c} Hilfsstoff wird während des Prozesses entfernt; Menge abhängig von Ansatzgröße | |

Die Lackierung der wirkstoffbeladenen Pellets erfolgt durch Aufsprühen einer Dispersion bestehend aus Ethylcellulose-Dispersion, HPMCAS, TEC und Wasser in einer Wirbelschichtanlage (mit Wurstereinsatz). Die lackierten Pellets werden anschließend bei Temperaturen von 40-90°C getempert, um die Lagerstabilität der Pelletformulierung zu verbessern. Die lackierten Pellets werden danach verkapselt.

### Beispiele 2 bis 19: einschichtige Matrixtabletten

| Einsatzstoffe | Beispiel 2 | Beispiel 3 | Beispiel 4 |
|---|---|---|---|
| | Menge in mg | Menge in mg | Menge in mg |
| Vardenafil HCl trihydrat mikronisiert^{a} | 23,7 | 23,7 | 23,7 |
| HPMC 100 | 150 | - | - |
| HPMC 400 | - | 150 | - |
| Hydroxypropylcellulose^{b} | - | - | 150 |
| Mikrokristalline Cellulose | 63,8 | 63,8 | 63,8 |
| Magnesiumstearat | 2,5 | 2,5 | 2,5 |

| | | | |
|---|---|---|---|
| ^{a} Wirkstoffmenge entsprechend 20 mg Vardenafil ^{b}Typ M | | | |

| **Einsatzstoffe** | Beispiel 5 | Beispiel 6 | Beispiel 7 |
|---|---|---|---|
| | Menge in mg | Menge in mg | Menge in mg |
| Vardenafil HCl trihydrat mikronisiert^{a} | 23,7 | 23,7 | 23,7 |
| HPCM 4000 | 130 | - | - |
| HPCM 15000 | - | 90 | - |
| HPCM 100000 | - | - | 80 |
| Mikrokristalline Cellulose | 59,5 | - | - |
| Lactose^{b} | - | 84,3 | 94,3 |
| Hochdisperses Siliciumdioxid | 2,0 | - | - |
| Magnesiumstearat | 4,8 | 2,0 | 2,0 |

| | | | |
|---|---|---|---|
| ^{a} Wirkstoffmenge entsprechend 20 mg Vardenafil ^{b} Tablettose 100 | | | |

### Herstellung Beispiel 2 bis 7:

Die Bestandteile der einschichtigen Matrix-Tabletten mit Ausnahme von Magnesiumstearat und ggf. Siliciumdioxid werden gemischt. Magnesiumstearat und ggf. Siliciumdioxid werden als Nachmischung zugemischt. Anschließend werden die Pulvermischung direkttablettiert (Format: rund 8 mm). Die erhaltenen Tabletten können lackiert oder überzogen werden, beispielsweise zur Gewährleistung von Lichtschutz oder zur Verzögerung bzw. Retardierung der Freisetzung.

| **Einsatzstoffe** | Beispiel 8 | Beispiel 9 | Beispiel 10 |
|---|---|---|---|
| | Menge in mg | Menge in mg | Menge in mg |
| Vardenafil HCl trihydrat mikronisiert^{a} | 23,7 | 23,7 | 23,7 |
| Methyacrylic acid copolymer | 100 | | - |
| Type C^{b} | | | |
| HPMCAS^{c} | - | 100 | - |
| Celluloseacetatphthalat (CAP) | - | - | 100 |
| HPMC 1500 | 123,3 | 123,3 | 123,3 |
| Mikrokristalline Cellulose | 50,0 | 50,0 | 50,0 |
| Magnesiumstearat | 3,0 | 3,0 | 3,0 |

| | | | |
|---|---|---|---|
| ^{a} Wirkstoffmenge entsprechend 20 mg Vardenafil ^{b} Eudragit^{®} L 100-55 ^{c} Hydroxypropylmethylcelluloseacetatsuccinat, Aqoat AS-LF | | | |

| **Einsatzstoffe** | Beispiel 11 | Beispiel 12 | Beispiel 13 |
|---|---|---|---|
| | Menge in mg | Menge in mg | Menge in mg |
| Vardenafil HCl trihydrat mikronisiert^{a} | 23,7 | 23,7 | 23,7 |
| Citronensäure | 100 | - | - |
| Bernsteinsäure | - | 100 | - |
| Weinsäure | - | - | 100 |
| HPMC 4000 | 130 | 130 | 130 |
| Mikrokristalline Cellulose | 59,5 | 59,5 | 59,5 |
| Hochdisperses Siliciumdioxid | 2,0 | 2,0 | 2,0 |
| Magnesiumstearat | 4,8 | 4,8 | 4,8 |

| | | | |
|---|---|---|---|
| ^{a} Wirkstoffmenge entsprechend 20 mg Vardenafil | | | |

| Einsatzstoffe | Beispiel 14 | Beispiel 15 | Beispiel 16 |
|---|---|---|---|
| | Menge in mg | Menge in mg | Menge in mg |
| Vardenafil mikronisiert | 20,0 | - | - |
| Vardenafil dihydrat mikronisiert^{a} | - | 21,5 | - |
| Vardenafil dimesylat monohydrat | - | - | 28,6 |
| mikronisiert^{a} | | | |
| HPCM 4000 | 110 | 110 | 115 |
| Weinsäure | 100 | 100 | 100 |
| Avicel | 63,0 | 63,0 | 79,9 |
| Hochdisperses Siliciumdioxid | 2,0 | 1,0 | 2,0 |
| Magnesiumstearat | 5,0 | 4,5 | 4,5 |

| | | | |
|---|---|---|---|
| ^{a} Wirkstoffmenge entsprechend 20 mg Vardenafil | | | |

| **Einsatzstoffe** | Beispiel 17 | Beispiel 18 | Beispiel 19 |
|---|---|---|---|
| | Menge in mg | Menge in mg | Menge in mg |
| Vardenafil HCl trihydrat mikronisiert^{a} | 11,85^{a} | 35,6^{b} | 47,4^{c} |
| Weinsäure | 70 | 150 | 150 |
| HPMC 4000 | 100 | 120 | 120 |
| Mikrokristalline Cellulose | 43,15 | 86,4 | 74,6 |
| Hochdisperses Siliciumdioxid | 1,5 | 2,0 | 2,0 |
| Magnesiumstearat | 3,5 | 6,0 | 6,0 |

| | | | |
|---|---|---|---|
| ^{a} Wirkstoffmenge entsprechend 10 mg Vardenafil ^{b} Wirkstoffmenge entsprechend 20 mg Vardenafil ^{c}Wirkstoffmenge entsprechend 30 mg Vardenafil | | | |

### Herstellung Beispiel 8 bis 19:

Die Bestandteile der einschichtigen Matrix-Tabletten mit Ausnahme von Magnesiumstearat und ggf. Siliciumdioxid werden gemischt. Anschließend wird die Mischung durch Walzenkompaktierung trocken granuliert und nach Zumischen von Siliciumdioxid und Magnesiumstearat tablettiert. Die erhaltenen Tabletten können lackiert oder überzogen werden, beispielsweise zur Gewährleistung von Lichtschutz oder zur Verzögerung bzw. Retardierung der Freisetzung.

### Beispiele 20 bis 25: 2-Schichttabletten

| Einsatzstoffe | Beispiel 20 | Beispiel 21 | Beispiel 22 |
|---|---|---|---|
| | Menge in mg | Menge in mg | Menge in mg |
| **Schnell freisetzende Schicht** | | | |
| Vardenafil HCl trihydrat mikronisiert^{a} | 11,85 | 11,85 | 11,85 |
| Mikrokristalline Cellulose | 105 | 105 | 105 |
| Crospovidone | 6,25 | 6,25 | 6,25 |
| Hochdisperses Siliciumdioxid | 0,63 | 0,63 | 0,63 |
| Magnesiumstearat | 1,25 | 1,25 | 1,25 |
| **Retard-Schicht** | | | |
| Vardenafil HCl trihydrat mikronisiert^{b} | 23,7 | 23,7 | 23,7 |
| Weinsäure | 100 | 100 | 100 |
| HPMC 50 | 30 | - | - |
| HPMC 4000 | 30 | 85 | 130,0 |
| Mikrokristalline Cellulose | 109,8 | 84,8 | 59,5 |
| Hochdisperses Siliciumdioxid | 2,0 | 2,0 | 2,0 |
| Magnesiumstearat | 4,5 | 4,5 | 4,8 |

| | | | |
|---|---|---|---|
| ^{a} Wirkstoffmenge entsprechend 10 mg Vardenafil ^{b} Wirkstoffmenge entsprechend 20 mg Vardenafil | | | |

| Einsatzstoffe | Beispiel 23 | Beispiel 24 | Beispiel 25 |
|---|---|---|---|
| | Menge in mg | Menge in mg | Menge in mg |
| **Schnell freisetzende Schicht** | | | |
| Vardenafil HCl trihydrat mikronisiert^{a} | 5,93 | 5,93 | 5,93 |
| Mikrokristalline Cellulose | 68,87 | 59,52 | 59,52 |
| Crospovidone | 4,0 | 3,5 | 3,5 |
| Hochdisperses Siliciumdioxid | 0,4 | 0,35 | 0,35 |
| Magnesiumstearat | 0,8 | 0,7 | 0,7 |
| **Retard-Schicht** | | | |
| Vardenafil HCl trihydrat mikronisiert^{b} | 11,85 | 11,85 | 11,85 |
| Weinsäure | 50 | 70 | 90 |
| HPMC 4000 | 100 | 100 | - |
| | | | |
| HPMC 15000 | - | - | 100 |
| Mikrokristalline Cellulose | 43,15 | 43,15 | 43,15 |
| Hochdisperses Siliciumdioxid | 1,5 | 2,0 | 2,0 |
| Magnesiumstearat | 3,5 | 4,5 | 4,8 |

| | | | |
|---|---|---|---|
| ^{a} Wirkstoffmenge entsprechend 5 mg Vardenafil ^{b} Wirkstoffmenge entsprechend 10 mg Vardenafil | | | |

Die Bestandteile der schnell freisetzenden Schicht mit Ausnahme der Nachmischung (Siliciumdioxid, mikrokristallinen Cellulose (ca. 15% der Gesamtmenge) sowie Magnesiumstearat) werden gemischt und durch Walzenkompaktierung granuliert. Ebenso werden die Komponenten der Retardschicht mit Ausnahme der Nachmischungskomponenten (Siliciumdioxid und Magnesiumstearat) gemischt und kompaktiert. Beide Granulate werden nach Zumischen der Nachmischung auf einer 2-Schicht-Tablettenpresse tablettiert (Format: rund 10 mm bei Beispiel 20 bis 22 und rund 9 mm bei Beispiel 23 bis 25). Die 2-Schicht-Tabletten können lackiert oder überzogen werden, beispielsweise zur Gewährung von Lichtschutz.

### Beispiele 26 bis 31: Schmelzextrudate

| **Einsatzstoffe** | Beispiel 26 | Beispiel 27 | Beispiel 28 |
|---|---|---|---|
| | Menge in mg | Menge in mg | Menge in mg |
| Vardenafil mikronisiert | 20 | 20 | 20 |
| Hydroxypropylcellulose^{a} | 180 | 180 | 320 |
| Methyacrylic acid copolymer | 140 | 140 | - |
| Type C^{a} | | | |
| Maltitol | 40 | - | - |
| Xylitol | - | 40 | 40 |
| Magnesiumstearat | 20 | 20 | 20 |

| | | | |
|---|---|---|---|
| ^{a} Eudragit^{®} L 100-55 | | | |

| | Beispiel 29 | Beispiel 30 | Beispiel 31 |
|---|---|---|---|
| | M | Menge in mg | Menge in mg |
| | enge in mg | | |
| Vardenafil mikronisiert | 20 | 20 | 20 |
| Hydroxypropylcellulose | 180 | 80 | 70 |
| HPMCASa | 160 | 70 | |
| Ammonio methacrylate copolymer | | | 70 |
| Type Bb | | | |
| Maltitol | 40 | - | - |
| Xylitol | - | 20 | - |
| Magnesiumstearat | 20 | 10 | 10 |
| Benzoesäure | - | - | 30 |

| | | | |
|---|---|---|---|
| a Aqoat AS-LF c Eudragit® RS PO | | | |

Der Wirkstoff wird mit den Hilfsstoffen für das Extrudat gemischt. Diese Mischung wird im Extruder bei einer geeigneten Temperatur extrudiert (z.B. 120-190°C). Durch Schneiden des Extrudatstranges in Stücke geeigneter Länge (ca. 2-3 mm) werden Pellets geformt. Anschließend können diese Schmelzextrusionspellets ausgerundet werden. Die Pellets können, um ein Zusammenkleben z.B. während der Freisetzung zu verhindern, mit einer Dispersion z.B. bestehend aus einer Ethyl acrylate methyl methacrylate Copolymer-Dispersion, HPMC, Polysorbat, Magnesiumstearat und Wasser in einem Wirbelschichtgranulator mit Wurstereinsatz lackiert werden. Schließlich können die Extrudate verkapselt werden.

### Beispiele 32 bis 34: Osmotische Systeme (zweischichtig)

| **Einsatzstoffe** | Beispiel 32 | Beispiel 33 | Beispiel 34 |
|---|---|---|---|
| | Menge in mg | Menge in mg | Menge in mg |
| **Wirkstoffschicht** | | | |
| Vardenafil mikronisiert | 22,0 | - | - |
| Vardenafil dihydrat mikronisiert^{a} | - | 23,7 | - |
| Vardenafil HCl trihydrat mikronisiert^{a} | - | - | 26,1 |
| HPMC5 | 5 | 555 | 5 |
| Polyethylenoxid | 101,5 | 99,8 | 97,4 |
| Hochdisperses Siliciumdioxid | 1,0 | 1,0 | 1,0 |
| Magnesiumstearat | 0,5 | 0,5 | 0,5 |
| **Osmotische Schicht** | | | |
| HPMC5 | 4 | 4 | 4 |
| Natriumchlorid | 25 | 25 | 25 |
| Polyethylenoxid | 55 | 55 | 55 |
| Eisenoxid rot | 0,8 | 0,8 | 0,8 |
| Magnesiumstearat | 0,2 | 0,2 | 0,2 |
| Osmose-Membran | | | |
| Celluloseacetat | 28,5 | 28,5 | 28,5 |
| Polyethylenglycol 3350 | 1,5 | 1,5 | 1,5 |

| | | | |
|---|---|---|---|
| ^{a} Wirkstoffmenge entsprechend 22 mg Vardenafil; inkl. 10% Überschuss, der nach vollständiger Freisetzung in Arzneiform verbleibt | | | |

Aus den Komponenten der Wirkstoff- und osmotischen Schicht werden durch Trockengranulation und Tablettierung 2-Schichttabletten hergestellt (Format: rund 8 mm). Diese Tabletten werden mit einer Mischung aus Celluloseacetat und Polyethylenglycol in acetonischer Lösung lackiert. Die Tabletten werden auf geeignete Weise angebohrt. Anschließend können die Tabletten noch überlackiert werden, z.B. mit einem Lichtschutzlack.

### Beispiele 35 bis 37: Osmotische Systeme (einschichtig)

| **Einsatzstoffe** | Beispiel 35 | Beispiel 36 | Beispiel 37 |
|---|---|---|---|
| | Menge in mg | Menge in mg | Menge in mg |
| **Tablette** | | | |
| Vardenafil mikronisiert | 24,0 | - | - |
| Vardenafil dihydrat mikronisiert^{a} | - | 25,8 | - |
| Vardenafil HCl trihydrat mikronisiert^{a} | - | - | 28,4 |
| Copovidone | 40 | 40 | 40 |
| Xanthan Gummi | 60 | 60 | 60 |
| Natriumcarboxymethylstärke TypA | 15 | 15 | 15 |
| Natriumchlorid | 38,5 | 36,7 | 34,1 |
| Natriumbicarbonat | 15 | 15 | 15 |
| HPMC5 | 5 | 555 | 5 |
| Natriumlaurylsulfat | 0,5 | 0,5 | 0,5 |
| Hochdisperses Siliciumdioxid | 1,0 | 1,0 | 1,0 |
| Magnesiumstearat | 1,0 | 1,0 | 1,0 |
| Osmose-Membran | | | |
| Celluloseacetat | 13,3 | 13,3 | 13,7 |
| Polyethylenglycol 3350 | 0,7 | 0,7 | 1,7 |

| | | | |
|---|---|---|---|
| ^{a} Wirkstoffmenge entsprechend 24 mg Vardenafil; inkl. 20% Überschuss, der nach vollständiger Freisetzung in Arzneiform verbleibt | | | |

Xanthan Gummi, Natriumchlorid, Natriumbicarbonat und Natriumcarboxymethylstärke und Copovidone werden gemischt und dann mit einer wässrigen Dispersion des Wirkstoffs mit HPMC und Natriumlaurylsulfat granuliert. Das Granulat wird mit Magnesiumstearat und kolloidalem Siliciumdioxid abgemischt und tablettiert (Format: rund 8 mm). Diese Tabletten werden mit einer Mischung aus Celluloseacetat und Polyethylenglycol in acetonischer Lösung lackiert. Die Tabletten werden auf geeignete Weise angebohrt. Anschließend können die Tabletten noch überlackiert werden, z.B. mit einem Lichtschutzlack.

### Beispiel 38: Freisetzungsuntersuchungen

Die Freisetzung aus zwei erfindungsgemäßen Formulierungen (Beispiel 5 und Beispiel 13) wird in der Blattrührer-Apparatur "Apparatur 2" des USP 28-NF 23 (The United States Pharmacopoeia USP 28 2005) bei einer Temperatur von 37 ± 0,5°C und einer Rührgeschwindigkeit von 75 UpM unter Verwendung von Sinkern in Medien mit verschiedenen pH-Werten untersucht. Als Freisetzungsmedien werden jeweils 900 ml 0,1 M Salzsäure (pH ca. 1,1), Acetatpuffer pH 4,5 nach USP (Herstellung von 1 Liter dieses Puffers: 2,99 g Natriumacetat trihydrat und 14 ml 2 N Essigsäure werden zu 1000 ml mit entmineralisiertem Wasser gelöst. Sofern erforderlich wird der pH-Wert mit Natriumhydroxid oder 2 N Essigsäure auf 4,5 ± 0,05 eingestellt) und Phosphatpuffer pH 6,8 mit 0,1% (m/V) Natriumlaurylsulfat (Herstellung von 1 Liter dieses Mediums: 2,747 g Dinatriumhydrogenorthophosphat dihydrat, 0,475 g Citronensäure monohydrat und 10 g Natriumlaurylsulfat-Lösung 10% (m/m) werden zu 1000 ml mit entionisiertem Wasser gelöst. Der pH-Wert wird mit Natriumhydroxid oder ortho-Phosphorsäure auf 6,8 ± 0,05 eingestellt) verwendet. Aus dem Freisetzungsmedium werden Proben durch eine Filtriereinheit entnommen, die gewährleisten muss, dass Begleitstoffe entfernt werden, und die darin gelöste Wirkstoffmenge durch HPLC mit UV-VIS-Detektion bestimmt. Die so bestimmte Wirkstoffmenge wird in Masse-Prozent der eingesetzten Wirkstoffmenge umgerechnet.

Die prozentuale Wirkstofffreisetzung über die Zeit ist in Abbildung 1 (Formulierung aus Beispiel 5) und Abb. 2 (Formulierungen aus Beispiel 13) dargestellt.

Die Abbildungen zeigen, dass es sich bei beiden Formulierungen um Darreichungsformen mit kontrollierter Wirkstofffreisetzung handelt, bei denen die oben definierte erfindungsgemäße Freisetzung erzielt wird. Die Formulierung aus Beispiel 13, deren Freisetzung in Abb. 2 dargestellt ist, stellt eine bevorzugte Ausführungsform der vorliegenden Erfindung dar, da bei dieser Darreichungsform durch Zusatz einer Säure die pH-Wert-Abhängigkeit gegenüber der Formulierung aus Beispiel 5 ohne pH-Wert modifizierende Zuschlagsstoffe, deren Freisetzung in Abb. 1 dargestellt ist, deutlich reduziert wird.

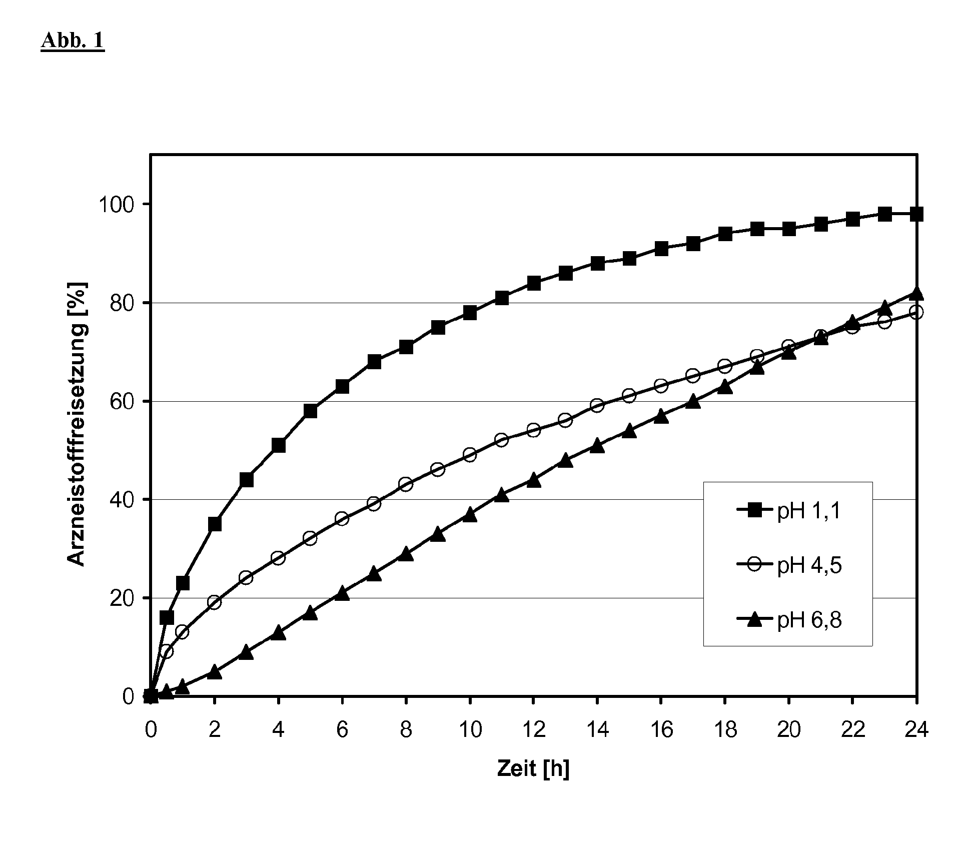

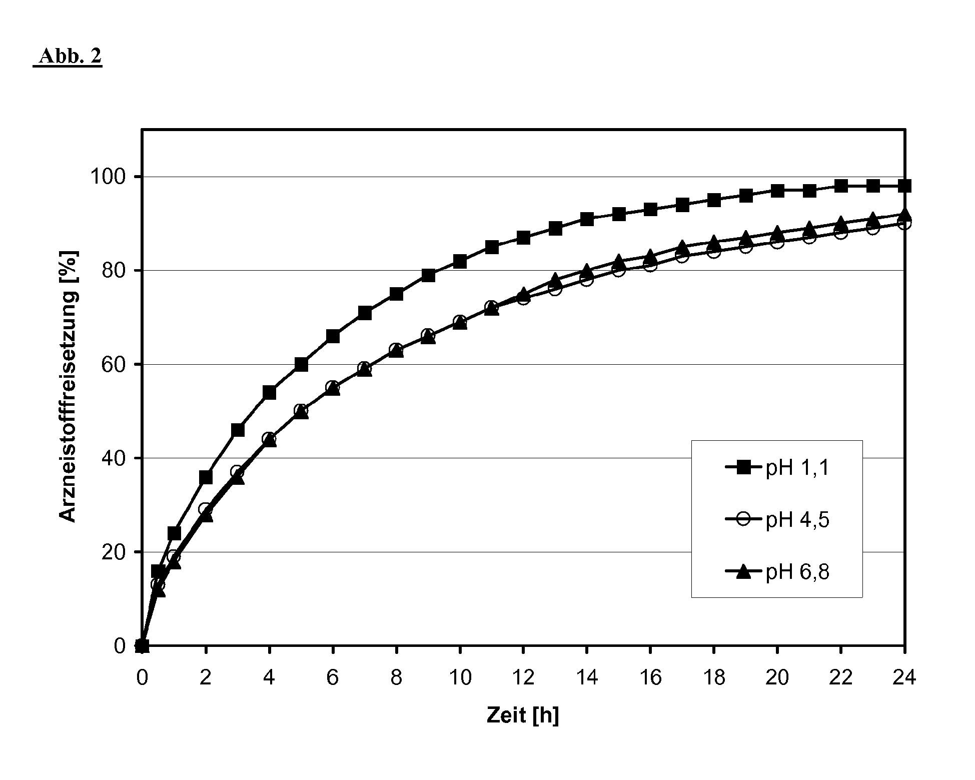

### Vergleichsbeispiel A und B:

| Einsatzstoffe | Vergleichsbeispiel 1 | Vergleichsbeispiel 2 |
|---|---|---|
| | Menge in mg | Menge in mg |
| Vardenafil HCl trihydrat mikronisiert^{a} | 23,7 | 23,7 |
| Hydroxypropylcellulose^{b} | 25 | - |
| HPMC 100000 | - | 150 |
| Weinsäure | 100 | - |
| Mikrokristalline Cellulose | 65,8 | 40,8 |
| Hochdisperses Siliciumdioxid | 2,0 | 2,0 |
| Magnesiumstearat | 3,5 | 3,5 |

| | | |
|---|---|---|
| ^{a} Wirkstoffmenge entsprechend 20 mg Vardenafil ^{b}Typ L | | |

### Herstellung Vergleichsbeispiele 1 und 2:

Die Bestandteile der Matrix-Tabletten mit Ausnahme von Siliciumdioxid und Magnesiumstearat werden gemischt. Siliciumdioxid und Magnesiumstearat werden als Nachmischung zugemischt. Anschließend werden die Pulvermischung direkttablettiert (Format: rund 8 mm).

Bei den Vergleichsbeispielen 1 und 2 handelt es sich um Formulierungen, die eine mittlere Freisetzungsrate von 80% in weniger als 2 Stunden (Vergleichsbeispiel 1) bzw. eine mittlere Freisetzungsrate von 80% in mehr als 24 Stunden (Vergleichsbeispiel 2) aufweisen. Diese nicht erfindungsgemäßen Formulierungen sind im Gegensatz zu den erfindungsgemäßen Darreichungsformen, die eine mittlere Freisetzungsrate zwischen 80% in 2 Stunden und 80% in 24 Stunden aufweisen, nicht geeignet, um die Probleme des Stands der Technik zu überwinden. So kann durch die zu hohe mittlere Freisetzungsrate von Vergleichsbeispiel 1 keine signifikante Verlängerung der Exposition und Wirkungsdauer im Vergleich zu Formulierungen des Stands der Technik erzielt werden. Weiterhin gelingt es nicht, durch Anwendung dieser Formulierung (Vergleichsbeispiel 1) konstante Blutspiegel zu erzielen und das Auftreten von Blutspiegelspitzen zu vermeiden. Dagegen führt die zu geringe mittlere Freisetzungsrate von Vergleichsbeispiel 2 zu einem gravierenden Verlust an Bioverfügbarkeit, so dass keine ausreichenden Blutspiegel erreicht werden, um die gewünschten klinischen Wirkungen zu erzielen.

**Vergleichsbeispiel 3 und 4:** Pharmakokinetische Parameter von Vardenafil nach oraler Applikation einer schnell freisetzenden Tablette entsprechend dem Stand der
Technik

| Parameter | Vergleichsbeispiel 3 | Vergleichsbeispiel 4 |
|---|---|---|
| | Dosis 10 mg | Dosis 20 mg |
| AUC [µg*h/L] | 28,8 | 70,0 |
| Cₘₐₓ [µg/L] | 7,03 | 18,5 |
| tₘₐₓ [h] | 0,88 | 0,99 |
| Mean residence time [h] | 5,00 | 4,69 |

**Beispiel 39 bis 41:** Pharmakokinetische Parameter von Vardenafil nach oraler Applikation von

erfindungsgemäßen Formulierungen mit kontrollierter Freisetzung und einer
niedrigen initialen Freisetzung (Dosis 20 mg)

| Parameter | Beispiel 39 | Beispiel 40 | Beispiel 41 |
|---|---|---|---|
| AUC [µg*h/L] | 78,8 | 64,4 | 64,5 |
| Cₘₐₓ [µg/L] | 7,93 | 5,05 | 4,30 |
| tₘₐₓ [h] | 4,00 | 6,00 | 6,00 |
| Mean residence time [h] | 8,68 | 11,6 | 12,4 |

**Beispiel 42 bis 44:** Pharmakokinetische Parameter von Vardenafil nach oraler Applikation von
erfindungsgemäßen Formulierungen mit kontrollierter Freisetzung und einer
hohen initialen Freisetzung (Dosis 30 mg)

| Parameter | Beispiel 42 | Beispiel 43 | Beispiel 44 |
|---|---|---|---|
| AUC [µg*h/L] | 119 | 105 | 108 |
| cₘₐₓ [µg/L] | 15,6 | 12,7 | 11,6 |
| tₘₐₓ [h] | 1,5 | 1,0 | 1,0 |
| Mean residence time [h] | 7,7 | 9,4 | 9,9 |

Die Beispiele zeigen, dass mit Hilfe der erfindungsgemäßen Darreichungsformen mit kontrollierter Wirkstofffreisetzung die Verweilzeit von Vardenafil im Körper signifikant verlängert wird im Vergleich zur Verweilzeit, die bei Applikation von dem Stand der Technik entsprechenden, schnell freisetzenden Formulierungen (Vergleichsbeispiel 3 und 4) resultiert. Die in Beispiel 39 bis 41 aufgeführten kinetischen Parameter von Vardenafil nach Applikation von erfindungsgemäßen Arzneimittelformulierungen mit einer relativ geringen initialen Freisetzung zeigen, dass mit derartigen Formulierungen nicht nur eine Verlängerung der MRT, sondern auch eine deutliche Reduktion von cₘₐₓ im Vergleich zur Applikation von schnell freisetzenden Formulierungen des Stands der Technik (Vergleichsbeispiel 3 und 4) erreicht werden kann. Diese Veränderungen der pharmakokinetischen Kenngrößen MRT und Cₘₐₓ werden ohne nennenswerte Reduzierung der AUC erreicht, d.h. die Bioverfügbarkeit der Formulierungen mit kontrollierter Wirkstofffreisetzung und einer relativ geringen initialen Freisetzung ist gegenüber einer schnell freisetzenden Formulierung kaum verändert. Beispiel 42 bis 44 dagegen zeigen die pharmakokinetischen Daten nach Applikation erfindungsgemäßer Formulierungen, die eine relativ hohe initiale Freisetzung besitzen. Mit Hilfe dieser Formulierungen kann ein schnelles Anfluten zum Erreichen eines gewünschten Blutspiegels erzielt und zusätzlich die mittlere Verweilzeit des Arzneistoffs im Körper im Vergleich zu Formulierungen des Stands der Technik (Vergleichsbeispiel 3 und 4) relevant verlängert werden. Auch für diese Formulierungen gilt, dass die Veränderung des pharmakokinetischen Profils bei praktisch (dosisnormiert) unveränderter AUC (= Bioverfügbarkeit) realisiert wird.

## Patentansprüche

1. Galenische Darreichungsform zur oralen Anwendung mit kontrollierter Wirkstofffreisetzung, die den PDE 5-Inhibitor Vardenafil und/oder pharmazeutisch verträgliche Salze und/oder Hydrate und/oder Solvate Hydrate der Salze und Solvate der Salze sowie der jeweils zugehörigen polymorphen, kristallinen und amorphen Formen davon als Wirkstoff enthalten, und die eine mittlere Freisetzungsrate zwischen 80% in 2 Stunden und 80% in 24 Stunden aufweist.

2. Galenische Darreichungsform nach Anspruch 1, **gekennzeichnet durch** einen Kern, der den Wirkstoff enthält und von einer Membran umschlossen wird, die die Freisetzung des Wirkstoffs kontrolliert und die freisetzungskontrollierende Membran ein filmbildendes Polymer und einen Weichmacher enthält.

3. Galenische Darreichungsform nach Anspruch 1 oder 2, **dadurch gekennzeichnet, dass** die freisetzungskontrollierende Membran ein filmbildendes Polymer und einen Porenbildner enthält.

4. Galenische Darreichungsform nach Anspruch 2, **gekennzeichnet dadurch, dass** der wirkstoffhaltige Kern eine pH-Wert modifizierende Substanz enthält.

5. Galenische Darreichungsform nach mindestens einem der Ansprüche 2 bis 4, **gekennzeichnet dadurch, dass** die freisetzungskontrollierende Membran ein magensaftresistentes Polymer enthält.

6. Galenische Darreichungsform nach mindestens einem der Ansprüche 1 bis 5, **gekennzeichnet dadurch, dass** sie den Wirkstoff in einer Matrix enthält, die den Wirkstoff durch Diffusion oder Erosion abgibt.

7. Galenische Darreichungsform nach Anspruch 6, **gekennzeichnet dadurch, dass** die Matrix ein wasserquellbares Polymer umfasst.

8. Galenische Darreichungsform nach mindestens einem der Ansprüche 6 bis 7, **gekennzeichnet dadurch, dass** die Matrix ein magensaftresistentes Polymer enthält.

9. Galenische Darreichungsform nach mindestens einem der Ansprüche 1 bis 7, **gekennzeichnet dadurch, dass** sie ein Schmelzextrudat des Wirkstoffs, das durch Einbettung des Wirkstoffs in eine Matrix mittels eines Schmelzprozesses hergestellt wird, enthält.

10. Galenische Darreichungsform nach Anspruch 9, **gekennzeichnet dadurch, dass** das Schmelzextrudat ein thermoplastisches Polymer umfasst.

11. Galenische Darreichungsform nach mindestens einem der Ansprüche 9 bis 10, **gekennzeichnet dadurch, dass** das Schmelzextrudat ein magensaftresistentes Polymer enthält.

12. Galenische Darreichungsform nach Anspruch 1, **dadurch gekennzeichnet, dass** es sich um eine osmotisches Arzneistofffreisetzungssystem, bestehend aus:
• einem Kern, der den Wirkstoff, ggf. ein hydrophiles polymeres Quellmittel und ggf.
einen wasserlöslichen Stoff zum Auslösen der Osmose sowie ggf. weitere pharmazeutisch akzeptable Hilfsstoffe enthält
• und einer Hülle, die aus einem wasserdurchlässigen, für die Komponenten des wirkstoffhaltigen Kerns undurchlässigen Material besteht und mindestens eine Öffnung aufweist, durch die im Kern vorhandene Bestandteile freigesetzt werden können.

13. Galenische Darreichungsform nach Anspruch 12, die Polyethylenoxide, Xanthan Gummi und/oder Copolymere aus Vinylpyrrolidon und Vinylacetat enthält.

14. Galenische Darreichungsform nach mindestens einem der Ansprüche 1 bis 13, die 1 bis 100 mg des Wirkstoffs berechnet als Vardenafil enthält.

15. Galenische Darreichungsform nach mindestens einem der Ansprüche 1 bis 14, die eine Matrix umfasst, die 1 bis 30% (m/m) Vardenafil hydrochlorid trihydrat, 10 bis 65% eines wasserlöslichen Polymers mit einer nominalen Viskosität von mindestens 50 cP und 10 bis 50% (m/m) einer organischen Säure bezogen auf die Gesamtmasse der Matrix enthält.
